# EUROPEAN PATENT SPECIFICATION

(11) **EP 3 152 225 B1**
(45) Date of publication and mention of the grant of the patent: **14.11.2018**
(21) Application number: 15725069.7
(22) Date of filing: 03.06.2015
(51) Int. Cl.: C07K 7/16, A61K 38/08

(54) **PEPTIDES AS OXYTOCIN AGONISTS**
PEPTIDE ALS OXYTOCINAGONISTEN
PEPTIDES UTILISÉS À TITRE D'AGONISTES D'OXYTOXINE

(30) Priority: 06.06.2014 EP 14171440
(43) Date of publication of application: 12.04.2017
(73) Proprietor: F. Hoffmann-La Roche AG, 4070 Basel (CH)
(72) Inventor: BISSANTZ, Caterina, 79102 Freiburg (DE); BLEICHER, Konrad, 79102 Freiburg (DE); CHAKRABORTY, Kanchan, West Bengal 721 305 (IN); GRUNDSCHOBER, Christophe, 4118 Rodersdorf (CH); SAHA, Goutam, Kolkata West Bengal 700 153 (IN)
(74) Representative: Salud, Carlos E.
(86) International application number: PCT/EP2015/062314
(87) International publication number: WO 2015/185584

(56) References cited:
- WO-A1-2009/122285
- WO-A2-2011/035330
- STYMIEST JAKE L ET AL: "Synthesis of biologically active dicarba analogues of the peptide hormone oxytocin using ring-closing metathesis", ORGANIC LETTERS, AMERICAN CHEMICAL SOCIETY, US, vol. 5, no. 1, 1 January 2003 (2003-01-01) , pages 47-49, XP009100775, ISSN: 1523-7060, DOI: 10.1021/OL027160V
- JAKE L STYMIEST ET AL: "Synthesis of Oxytocin Analogues with Replacement of Sulfur by Carbon Gives Potent Antagonists with Increased Stability", THE JOURNAL OF ORGANIC CHEMISTRY, AMERICAN CHEMICAL SOCIETY, US, vol. 70, no. 20, 30 September 2005 (2005-09-30), pages 7799-7809, XP002669141, ISSN: 0022-3263, DOI: 10.1021/JO050539L - [retrieved on 2005-04-30]

## Description

The invention relates to compounds of formula 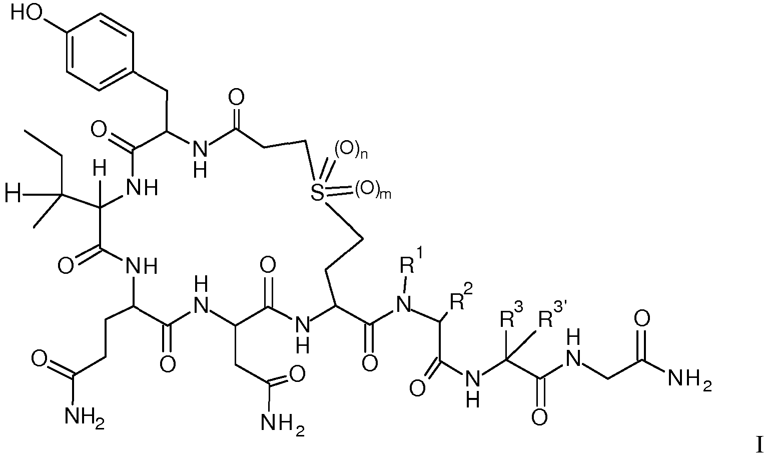 wherein
- R¹: is hydrogen, C₁₋₇-alkyl -CH₂-C₃₋₆-cycloalkyl or C₃₋₆-cycloalkyl;
- R²: is hydrogen, C₁₋₇-alkyl, C₁₋₇-alkyl substituted by hydroxy or
- R¹ and R²: may form together with the N and C atom to which they are attached a pyrrolidine ring optionally substituted by one or two F-atoms or by hydroxy, or may form an azetidine or a piperidine ring;
- R³: is hydrogen, C₁₋₇-alkyl, C₁₋₇-alkyl substituted by hydroxy, -(CH₂)ₒNH₂, benzyl optionally substituted by hydroxy, phenyl, -CH₂-C₃₋₆-cycloalkyl or C₃₋₆-cycloalkyl;
- R^{3'}: is hydrogen or C₁₋₇-alkyl;
- n: is 1;
- m: is 0 or 1
- o: is 1 to 4;
or to pharmaceutically acceptable acid addition salt, to a racemic mixture or to its corresponding enantiomer and/or optical isomers thereof.

It has been found that the present compounds are oxytocin receptor agonists, which compounds are oxytocin analogs that retain oxytocin bioactivity. Such analog molecules are capable of acting in a manner similar to endogenous oxytocin, including binding the oxytocin receptor. Analogs of oxytocin have completely new molecular structures.

Oxytocin is a nine amino acid cyclic peptide hormone with two cysteine residues that form a disulfide bridge between position 1 and 6. Human oxytocin comprises the sequence Cys-Tyr-Ile-Gln-Asn-Cys-Pro-Leu-Gly.

Peptides have emerged as a commercially relevant class of drugs that offer the advantage of greater specifity and potency and lower toxicity profiles over traditional small molecule pharmaceuticals. They offer promising treatment options for numerous diseases, such as diabetes, HIV, hepatitis, cancer and others, with physicians and patents becoming more accepting of peptide-based medicines. The present invention relates to peptidic oxytocin receptor agonists, which also include the natural hormone oxytocin and carbetocin.
Oxytocin is a potent uterotonic agent for the control of uterine atony and excessive bleeding, clinically used to induce labour, and has been shown to enhance the onset and maintenance of lactation (Gimpl et al., Physiol. Rev., 81, (2001), 629 - 683, Ruis et al.,BMJ, 283, (1981), 340 - 342). Carbetocin (1-deamino-1-carba-2-tyrosine (O-methyl)-oxytocin) is also a potent uterotonic agent clinically used for the control of uterine atony and excessive bleeding.

Peptidic oxytocin agonists may be used for the treatment of Prader-Willi Syndrom, which is a rare genetic disorder which affects one child in 25.000.
Further research indicates that oxytocin agonists are useful for the treatment of inflammation and pain, including abdominal and back pain (Yang, Spine, 19, 1994, 867-71), sexual dysfunction in both male (Lidberg et al., Pharmakopsychiat., 10, 1977, 21 - 25) and female (Anderson-Hunt, et al., BMJ, 309, 1994, 929), irretable bowel syndrome (IBS, Louvel et al., Gut, 39, 1996, 741 - 47), constipation and gastrointestinal obstruction (Ohlsson et al., Neurogastroenterol. Motil., 17, 2005, 697 - 704), autism (Hollander et al., Neuropsychopharm., 28, 2008, 193 - 98), stress, including post traumatic stress disorder (PTSD) (Pitman et al., Psychiatry Research, 48, 107 - 117), anxiety, including anxiety disorders and depression (Kirsch et al., J. Neurosci., 25, 49, 11489 - 93, Waldherr et al., PNAS, 104, 2007, 16681 - 84), surgical blood loss or control of post-partum haemorrhage (Fujimoto et al., Acta Obstet. Gynecol., 85, 2006, 1310 -14), labor induction and maintenance (Flamm et al., Obstet. Gynecol., 70, 1987, 70 - 12), wound healing and infection, mastitis and placenta delivery, and osteoporosis. Additionally, oxytocin agonists may be useful for the diagnosis of both cancer and placental insufficiency.

Furthermore, the Articles "Intranasal Oxytocin blocks alcohol withdrawal in human subjects" (Alcohol Clin Exp Res, Vol, No. 2012) and "Breaking the loop: Oxytocin as a potential treatment for drug addiction" (Hormones and Behavior, 61, 2012 , 331- 339) propose to treat alcohol withdrawel and drug addiction with a oxytocin agonist.

Oxytocin and its receptors exists in areas of the brain implicated in the symptoms of schizophrenia, such as the nucleus accumbens and the hippocampus. The oxytocin receptor agonists may be used for the treatment of autism, stress, including post traumatic stress disorder, anxiety, including anxiety disorders and depression, schizophrenia, Alzheimer's disease, psychiatric disorders, memory loss and metabolic diseases (WO2012/016229).

Objects of the present invention are novel compounds of formula I and the use of compounds of formula I and their pharmaceutically acceptable salts for the treatment of CNS diseases related to the oxytocin receptor, which diseases are autism, stress, including post traumatic stress disorder, anxiety, including anxiety disorders and depression, schizophrenia, psychiatric disorders and memory loss, alcohol withdrawel,drug addiction and for the treatment of Prader-Willi Syndrom.
Further objects are the preparation of novel compounds of formula I and medicaments, containing them.

The present invention may provide selective, efficacious compounds, providing alternatives and/or improvements in the treatment of certain CNS diseases including autism, stress, including post traumatic stress disorder, anxiety, including anxiety disorders and depression, schizophrenia, psychiatric disorders and memory loss, alcohol withdrawel, drug addiction and for the treatment of Prader-Willi Syndrom..

It has been shown that the present peptides have a very good selectivity to the vasopressin receptors V1a and V2 as shown in the table. This may have a major advantage for use as medicament to avoid side effects. These physiological effects may be considered to be undesirable side effects in the case of medicines aimed at treating diseases of the central nervous system. Therefore it is desirable to obtain medicines having selectivity for the oxytocin receptor vs vasopressin receptor.

As used herein, the term "lower alkyl" denotes a saturated straight- or branched chain group containing from 1 to 7 carbon atoms, for example, methyl, ethyl, propyl, isopropyl, n-butyl, i-butyl, 2-butyl, t-butyl and the like.

The term "lower alkyl substituted by hydroxy" denotes a lower alkyl group as defined above, wherein at least one hydrogen atom is replaced by a hydroxy group.

The term "cycloalkyl" denotes a cyclic alkyl chain, containing from 3 to 6 carbon atoms.

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methanesulfonic acid, p-toluenesulfonic acid and the like.
Preferred are compounds of formula I, wherein m is 1.

One object of the present invention are compounds, wherein R¹ is hydrogen, lower alkyl, -CH₂-cycloalkyl or cycloalkyl and R² is hydrogen, lower alkyl, lower alkyl substituted by hydroxy, and the other definitions are as described above.

One further object of the present invention are compounds, wherein R¹ and R² may form together with the N and C atom to which they are attached a pyrrolidine ring optionally substituted by one or two F-atoms or by hydroxy, or may form an azetidine or a piperidine ring, and the other definitions are as described above.

The following specific compounds have been prepared and tested for their agonistic activity on the oxytocin receptor:

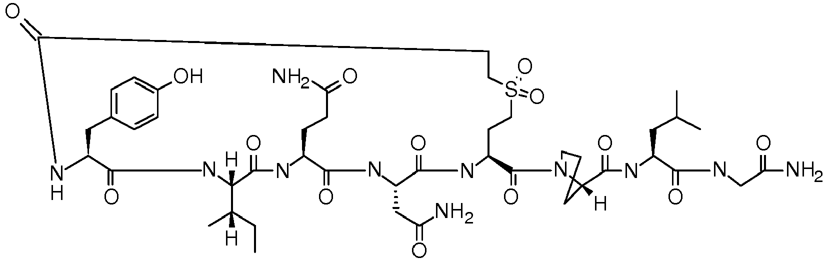

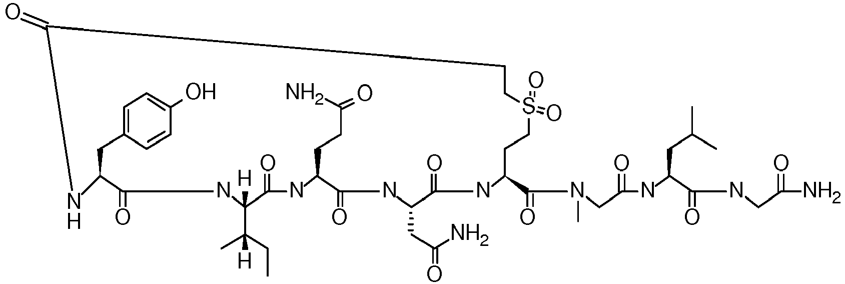

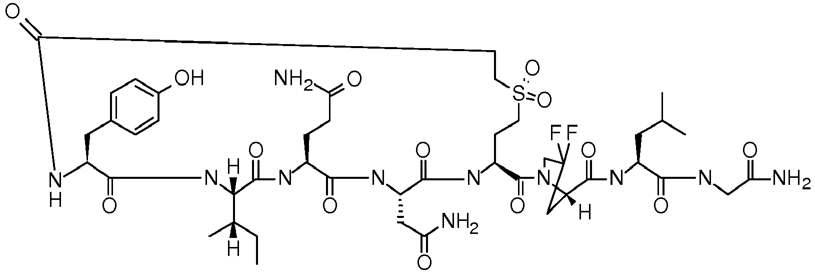

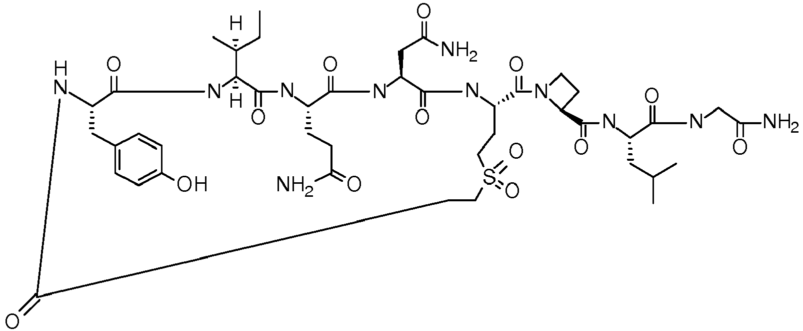

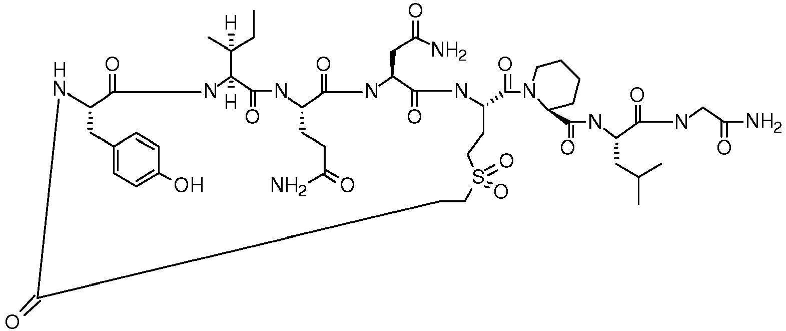

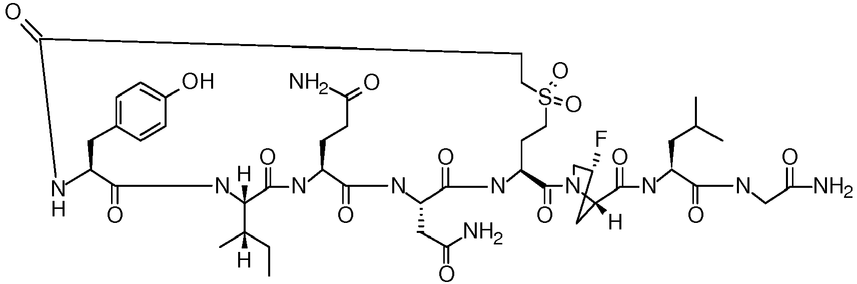

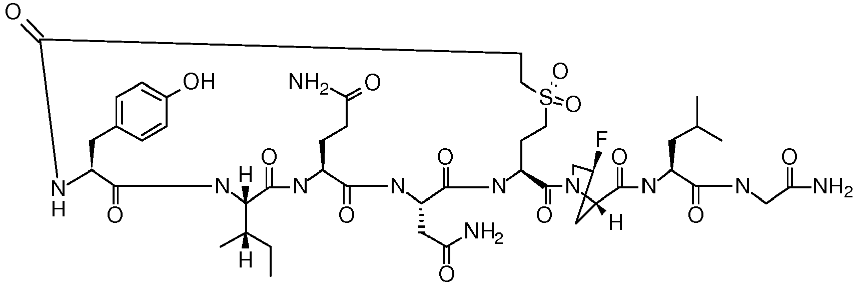

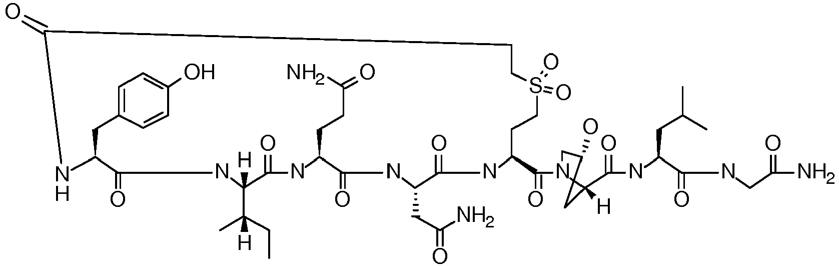

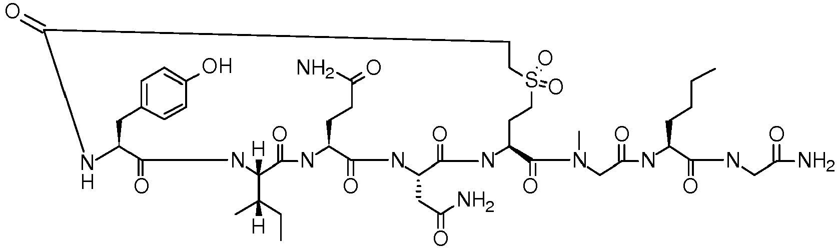

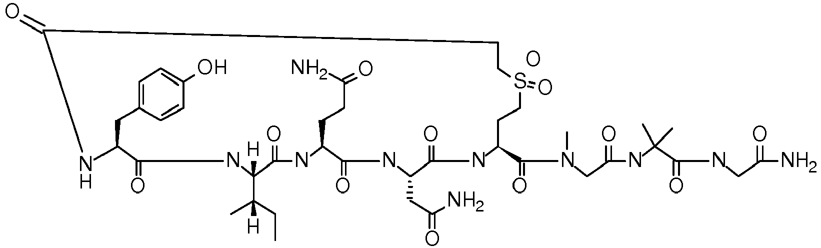

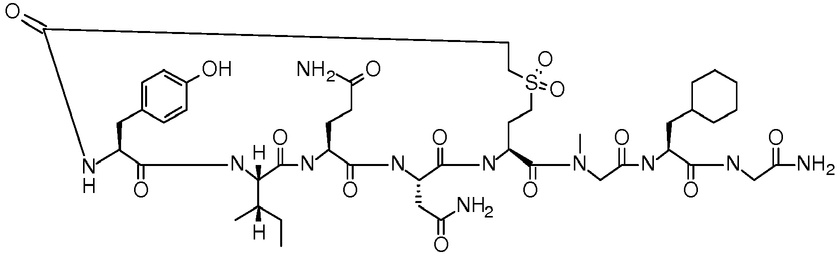

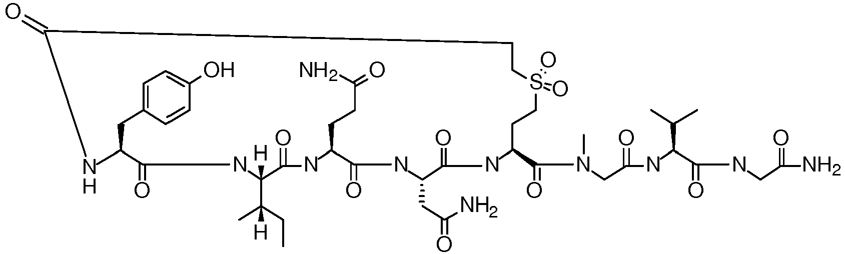

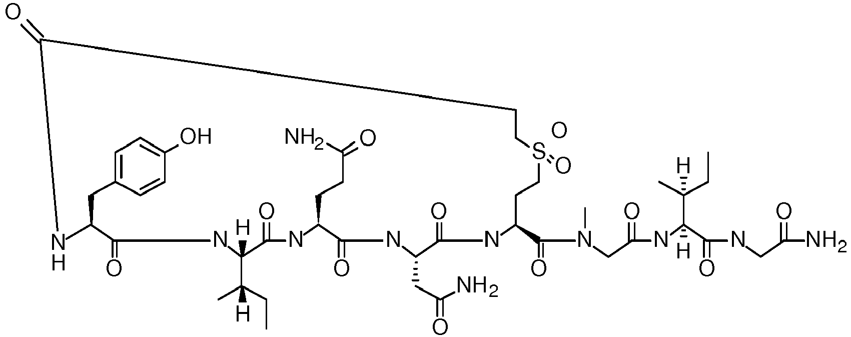

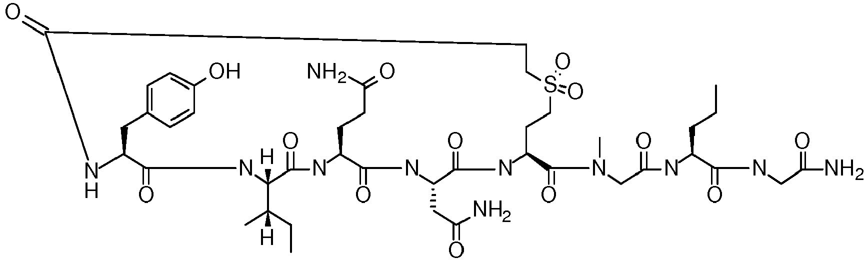

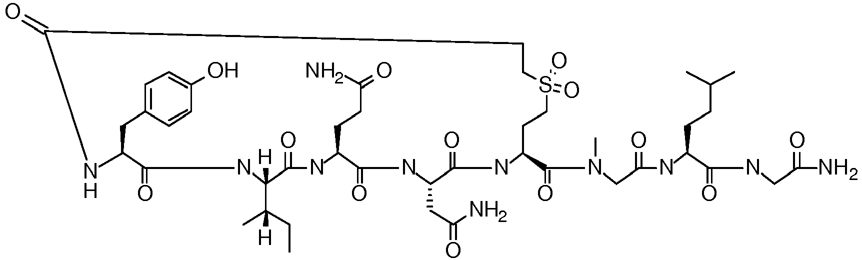

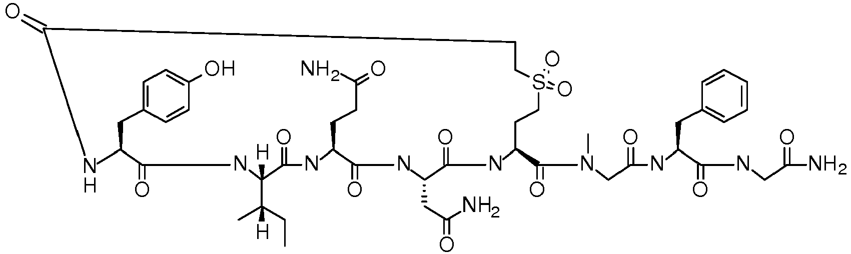

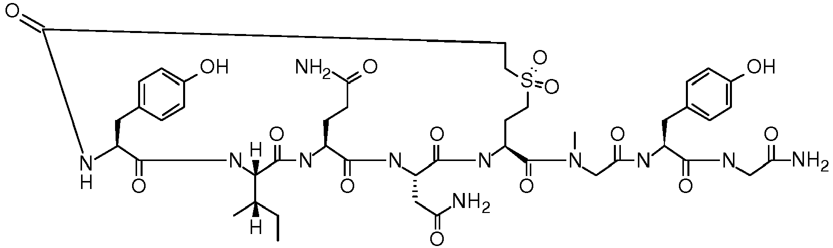

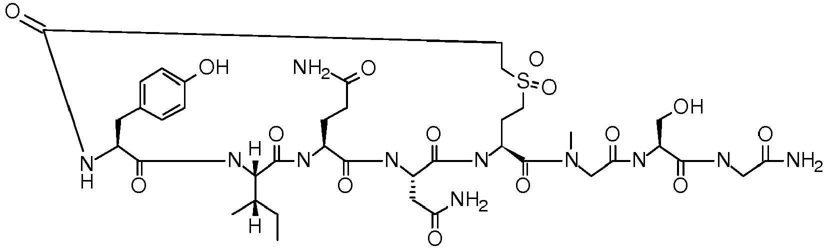

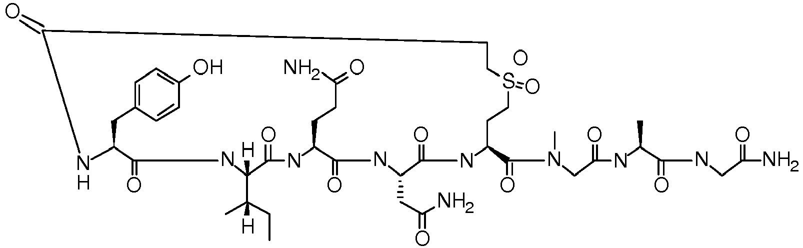

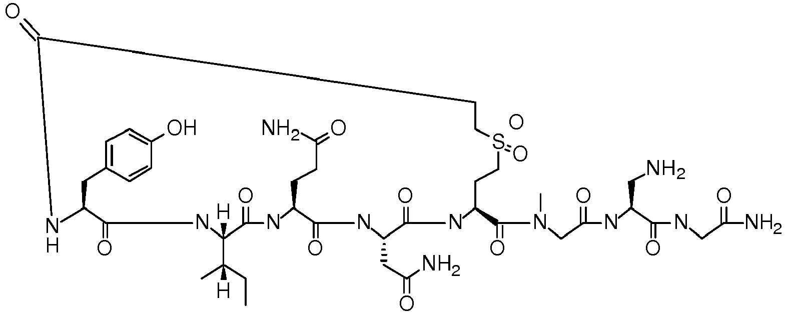

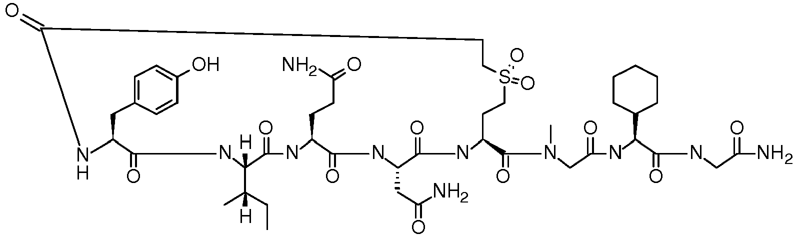

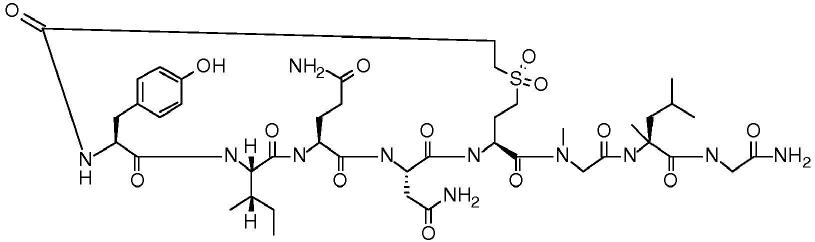

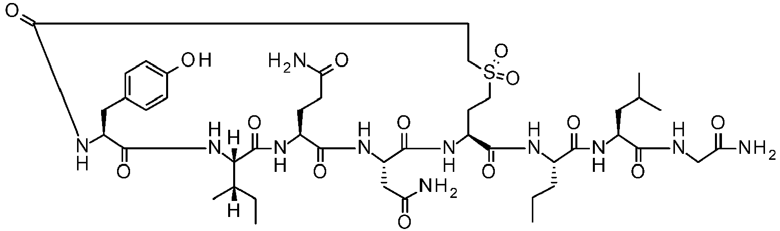

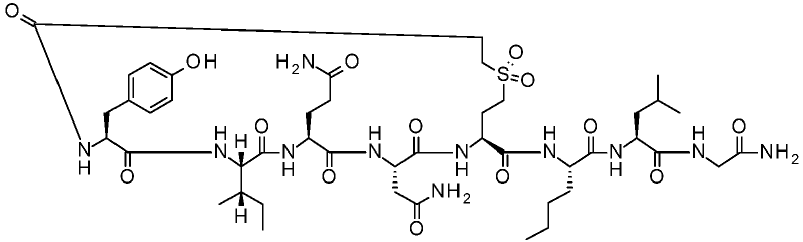

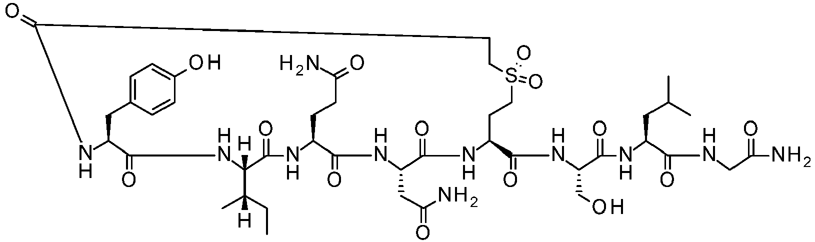

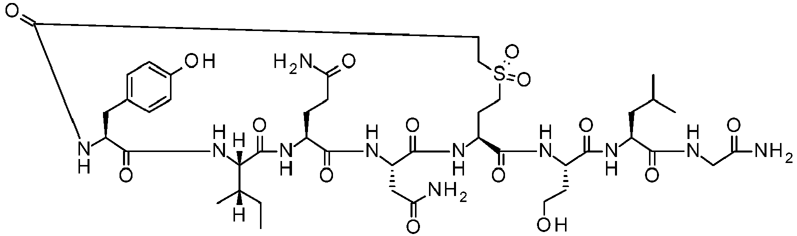

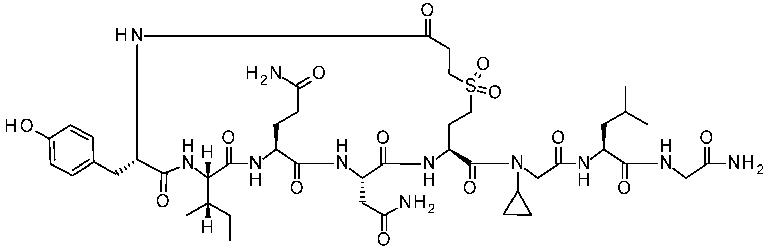

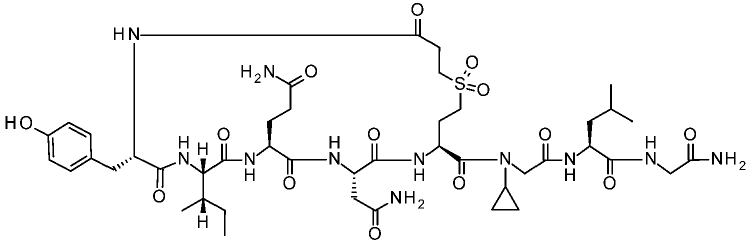

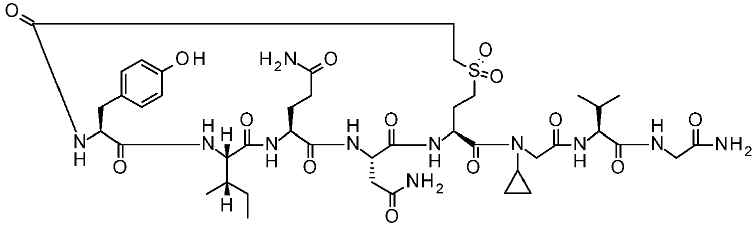

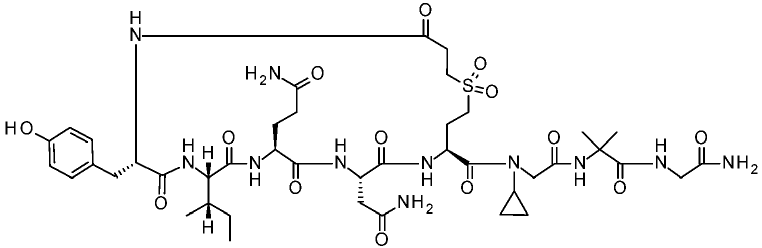

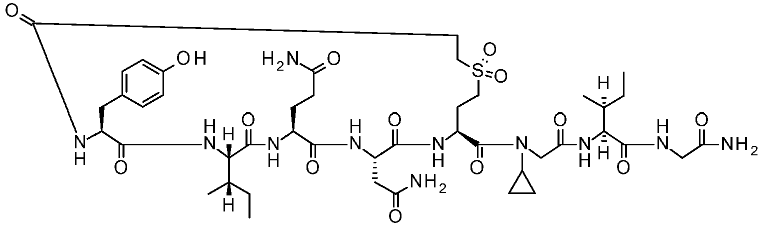

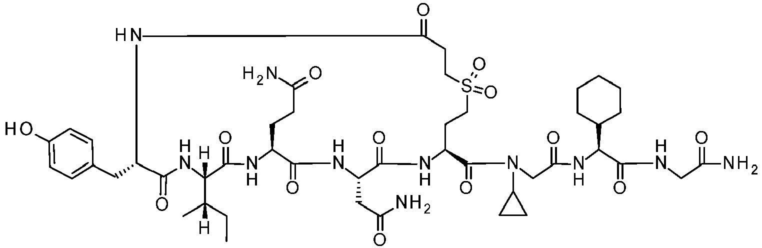

The preparation of compounds of formula **I** of the present invention may be carried out in sequential or convergent synthetic routes. The skills required for carrying out the reaction and purification of the resulting products are known to those skilled in the art.

The compounds herein were synthesized by standard methods in solid phase peptide chemistry utilizing both Fmoc and Boc methodology. Reactions carried out manually were performed at room temperature, while microwave assisted peptide synthesis was performed at elevated temperature.

### General Synthesis Description:

Linear peptides were either synthesized manually or using microwave technology via state-of-the-art solid phase synthesis protocols (Fmoc-chemistry) as referenced by e.g.: Kates and Albericio, Eds., "Solid Phase Synthesis: A practical guide", Marcel Decker, New York, Basel, 2000. As a solid support TentaGel-S-RAM resin (0.24 meq /g) was used. All Fmoc-amino acids were added in a 4-fold excess after activation with COMU (0.5 mol/L in DMF) and 4 eq of DIPEA (2 mol/L in NMP). Fmoc-cleavage was achieved with 20 % piperidine in DMF. Peptides were cyclized in solution after de-protection and cleavage from the resin and standard work-up. Crude peptides were treated with standard peptide activation regents in DMF. The cyclisation was monitored via HPLC.

### Cleavage & work-up:

A cleavage-cocktail of trifluoroacetic acid, triisopropylsilane and water (95/2.5/2.5) was added to the resin and shaken for 1h at RT. Cleaved peptides were precipitated in cold ether (-18 °C). The peptides were centrifuged and the residue washed twice with cold ether. The residues were again dissolved in water/ acetonitrile and lyophilized.

### Purification :

Peptides were purified using reversed phase high performance liquid chromatography (RP-HPLC) using a Reprospher 100 C18-T Colum (100 x 4.6 mm, 5um particle size) as a stationary phase and water/acetonitrile as eluent.

(Gradient 1-50 % MeCN over 30 min). Fractions were collected and analyzed by LC/MS. Pure product samples were combined and lyophilized. All peptides were obtained as white powders with a purity >85 %. Product identification was obtained via mass spectrometry.

All standard amino acids were purchased from CEM. (2S)-Fmoc-4,4-Difluoro-Pyrrolidine-2-Carboxylic Acid, Fmoc-Trans-4-Fluoro-Proline-OH, Fmoc-Hyp(tBu)-OH (2S,4S)-Fmoc-4-Fluoro-Pyrrolidine-2-Carboxylic Acid were purchased from Polypeptide. Fmoc-(S)-2-Amino-4-(2-tert-butoxycarbonyl-ethanesulfonyl)-butyric acid was generated as described below.

### Synthesis of Fmoc-(S)-2-Amino-4-(2-tert-butoxycarbonyl-ethanesulfonyl)-butyric acid

To a stirred solution of (2S)-2-amino-4-{[(3S)-3-amino-3-carboxypropyl]disulfanyl}butanoic acid (10 g, 37.263 mmol) in MeOH (250mL) was added SOCl₂ (10.8 mL, 149.05 mmol) drop wise over 20 min and reaction mixture was stirred at 25 °C for 18 h. The reaction mixture was then concentrated under reduced pressure to get methyl (2S)-2-amino-4-{[(3S)-3-amino-4-methoxy-4-oxobutyl] disulfanyl butanoate HCl salt (12 g, 87 %) as an off white solid. To a stirred suspension of methyl (2S)-2-amino-4-{[(3S)-3-amino-4-methoxy-4-oxobutyl]disulfanyl} butanoate HCl salt (24g, 64.97mmol) in H₂O(560 mL) were added K₂CO₃ (53.7 g, 389.8 mmol), Fmoc-Cl (33.6 g, 129.9 mmol) in dioxane (1800 mL) and reaction mixture was stirred at 25 °C for 18 h. The solid was filtered off, washed with MeOH (800 mL) and dried under reduced pressure to get methyl (2S)-2-{[(9H-fluoren-9-ylmethoxy)carbonyl]amino}-4-{[(3S)-3-{[(9H-fluoren-9-ylmethoxy)carbonyl]amino}-4-methoxy-4-oxobutyl]disulfanyl}butanoate (32 g, 66 %) as an off white solid. LCMS: 741 (M+H). To a stirred solution of methyl (2S)-2-{[(9H-fluoren-9-ylmethoxy)carbonyl]amino}-4-{[(3S)-3-{[(9H-fluoren-9-ylmethoxy)carbonyl]amino}-4-methoxy-4-oxobutyl]disulfanyl}butanoate, (16g, 21.6 mmol) in MeOH (864 mL) and DCM (240 mL) were added Zn dust (4.2 g, 64.8 mmol), TFA (64.3 mL, 863.8 mmol) and reaction mixture was stirred at 25 °C for 18 h. The reaction mixture was filtered to remove the zinc and the filtrate was concentrated under reduced pressure. The crude was taken up in ethyl acetate and washed with 1N HCl solution, 1N NaOH solution, water and brine solution. The separated organic layer was dried over sodium sulfate and evaporated under reduced pressure to get methyl (2S)-2-{[(9H-fluoren-9-ylmethoxy)carbonyl]amino}-4-sulfanylbutanoate which was directly used for next step without further purification. LC-MS: 372(M+H). To a stirred solution of methyl (2S)-2-{[(9H-fluoren-9-ylmethoxy)carbonyl]amino}-4-sulfanylbutanoate (8 g, 21.5 mmol) in MeOH/THF(2:1, 336 mL) were added triethylamine (3 mL, 21.5 mmol), tert-butyl prop-2-enoate (4.70 mL, 32.3 mmol) and reaction mixture was stirred at 25 °C for 3 h. The reaction mixture was evaporated under reduced pressure. The crude was taken up in DCM, washed with IN HCl, saturated NaHCO₃ solution, water and brine solution. The separated organic layer was dried over sodium sulfate and evaporated under reduced pressure. The crude thus obtained was purified by normal silica column using DCM (100 %) to get methyl (2S)-4-{[3-(tert-butoxy)-3-oxopropyl]sulfanyl}-2-{[(9H-fluoren-9-ylmethoxy)carbonyl]amino}butanoate (4.6 g, 44 %) as a colorless, sticky liquid. LC-MS: 500(M+H). To a stirred solution of methyl (2S)-4-{[3-(tert-butoxy)-3-oxopropyl]sulfanyl}-2-{[(9H-fluoren-9-ylmethoxy)carbonyl]amino} butanoate (4.6 g, 9.2 mmol) in isopropanol (122 mL) and H₂O (46 mL) were added CaCl₂(16.5 g, 149.2 mmol), LiOH (1.5 g, 36.8 mmol) and reaction mixture was stirred at 25 °C for 40 min. The organic solvents were removed under reduced pressure. The resulting residue was diluted with 10 % K₂CO₃ solution and washed with diethyl ether. The separated aqueous layer was acidified to pH∼2 with concentrated HCl and extracted with DCM. The separated organic layer was washed with water, brine solution, dried over anhydrous sodium sulfate and evaporated under reduced pressure to get (2S)-4-{[3-(tert-butoxy)-3-oxopropyl]sulfanyl}-2-{[(9H-fluoren-9-ylmethoxy)carbonyl]amino}butanoic acid (4g, 89 %) as an off white sticky solid. LC-MS: 484(M-H). To a stirred solution of (2S)-4-{[3-(tert-butoxy)-3-oxopropyl]sulfanyl}-2-{[(9H-fluoren-9-ylmethoxy)carbonyl] amino }butanoic acid (3.5 g, 7.2 mmol) in ethyl acetate (18 mL) was added oxone (22.15 g, 36.0 mmol) and reaction mixture was stirred at 25 °C for 48 h. Then reaction mass was filtered, solid was washed with ethyl acetate and filtrate evaporated under reduced pressure. The crude thus obtained was purified by normal silica column using 0-5 % MeOH in DCM to get (2S)-4-{[3-(tert-butoxy)-3-oxopropane]sulfonyl}-2-{[(9H-fluoren-9-ylmethoxy)carbonyl] amino }butanoic acid (3.1 g, 84 %) as a white solid. LC-MS: 516 (M-H).

### Peptide Synthesis:

The peptide was synthesized using CEM Microwave technology with coupling times of 5 minutes per amino acid at elevated temperature (78 °C) and a 0.25mmol scale. The synthesis is carried out using the TentalGel-S RAM resin as a solid support (0.24 meq /g). All amino acids used were dissolved in NMP to 0.2 mol concentration. A solution of 4 eq. COMU in DMF (0.5 mol/L) and DIPEA was used to activate the amino acids. Fmoc-Cleavage was achieved with Piperidine in DMF (20 %) for 3 min. Fmoc-cleavage was repeated.

### Cleavage from resin:

10ml of a cleavage-cocktail consisting of 95/2.5/2.5 Trifluoroacetic acid, Triisopropylsilane, and water was added to the resin and shaken for 3h at RT. Cleaved peptide was precipitated in cold Et₂O (-18 °C). The peptide was centrifuged 2 x 50 ml polypropylene tubes. The precipitates were washed two times with cold ether. Afterwards the precipitate was dissolved in H₂O/Acetonitrile and lyophilizied to yield 88 mg white powder.

### Cyclization :

Crude peptide was dissolved in DMF (15 ml). 1eq of coupling reagents PyoAP (0.5 mol/L) in DMF and DIPEA in NMP (2 mol/l) were added. The reaction mixture was stirred at RT for 1h. After the reaction was completed (LCMS control) the DMF content was concentrated down to approximately 2 ml. The residue was precipitated in cold (-18 °C) diethyl ether (40ml). The peptide was centrifuged and the precipitate washed with cold ether.

### Purification:

The crude peptide was purified by preparative HPLC on a Reprospher 100 C18-T Columm (100 x 4.6 mm, 5um particle size). As eluent system a mixture of 0.1 % TFA/water/acetonitrile was used with a gradient of 0-100 % acetonitrile within 0-75 min. The fractions were collected and checked by analytical HPLC. Fractions containing pure product were combined and lyophilized. 27 mg of white powder were obtained.

All other peptides listed below were synthesized accordingly.

### Abbreviations:

Fmoc: 9-Fluorenylmethoxycarbonyl
tBu: tert. Butyl
Gly: Glycine
Phe: Phenylalanine
Cha: Cyclohexylalanine
Chg: Cyclohexylglycine
Sar: Sarcosine
Hyp: Hydroxyproline
Ile: Isoleucine
Leu: Leucine
Nle: Norleucin
Nva: Norvaline
Dap: Diaminopropionic Acid
Aib: Aminoisobutyric Acid
Pro: Proline
Ala: Alanine
Val: Valine
homoVal: Homovaline
His(Trt): sidechain-protected (Trityl) Histidine
Asn(Trt): sidechain-protected (Trityl) Asparagine
Gln(Trt): sidechain-protected (Trityl) Glutamine
Tyr(tBu): sidechain-protected (tBu) Tyrosine
Thr(tBu): sidechain-protected (tBu) Threonine
HOBT: N-Hydroxybenzotriazole
COMU: 1-[(1-(Cyano-2-ethoxy-2-oxoethylideneaminooxy) dimethylaminomorpholino)] uronium hexafluorophosphate
PyoAP: (7-Azabenzotriazol-lyloxy) tripyrrolidino-phosphonium hexaflourophosphate
HBTU: O-Benzotriazole-N,N,N',N'-tetramethyl-uronium-hexafluorophosphate
DMF: N,N-Dimethylformamide
NMP: N-Methylpyrrolidone
DIPEA: N,N-Diisopropylamine
DCM: Dichlormethane
MeCN: Acetonitril

### Example 1

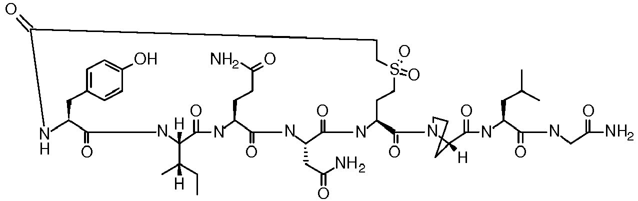

The following amino acids were used: Fmoc-Gly-OH, Fmoc-Leu-OH, Fmoc-Pro-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1006.1; observed 1006.4

### Example 2

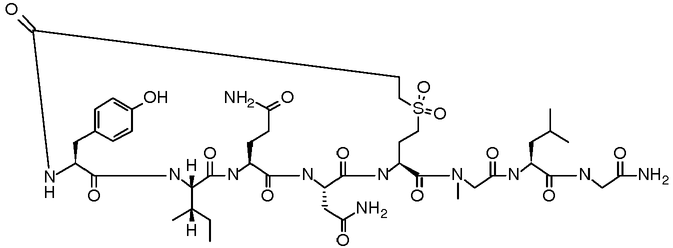

The following amino acids were used: Fmoc-Gly-OH, Fmoc-Leu-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 980.1; observed 981.2

### Example 3

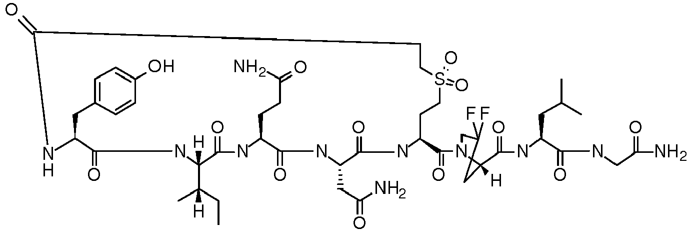

The following amino acids were used: Fmoc-Gly-OH, Fmoc-Leu-OH, (2S)-Fmoc-4,4-Difluoro-Pyrrolidine-2-Carboxylic Acid, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1042.1; observed 1043.1

### Example 4

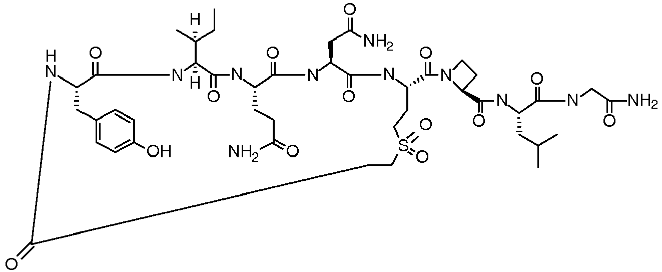

The following amino acids were used: Fmoc-Gly-OH, Fmoc-Leu-OH, (S)-N-Fmoc-Azetidine-2-Carboxylic Acid, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 992.2; observed 993.4

### Example 5

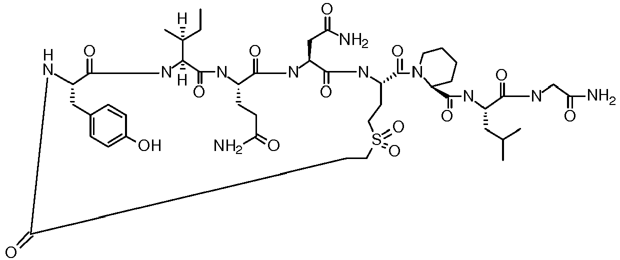

The following amino acids were used: Fmoc-Gly-OH, Fmoc-Leu-OH, Fmoc-Pipecolic Acid, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1020.2; observed 1020.5

### Example 6

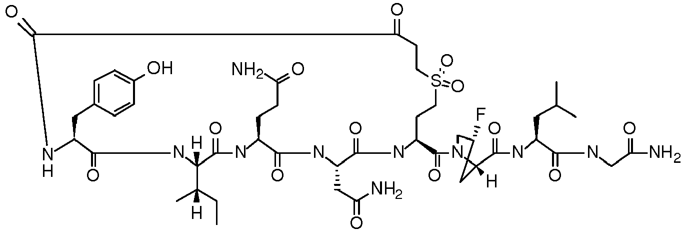

The following amino acids were used: Fmoc-Gly-OH, Fmoc-Leu-OH, (2S,4S)-Fmoc-4-Fluoro-Pyrrolidine-2-Carboxylic Acid, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1024.1; observed 1024.5

### Example 7

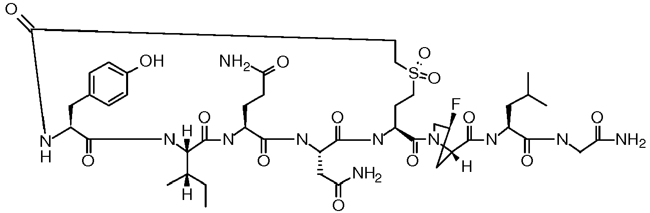

The following amino acids were used: Fmoc-Gly-OH, Fmoc-Leu-OH, Fmoc-Trans-4-Fluoro-Proline, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1024.1; observed 1024.5

### Example 8

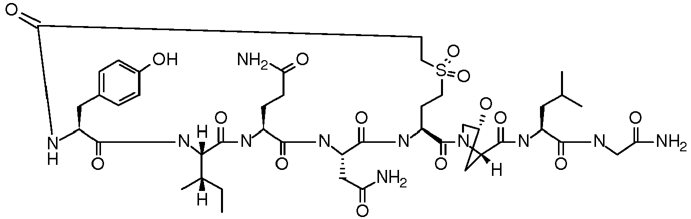

The following amino acids were used: Fmoc-Gly-OH, Fmoc-Leu-OH, Fmoc-Hyp(tBu)-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1022.1; observed 1022.5

### Example 9

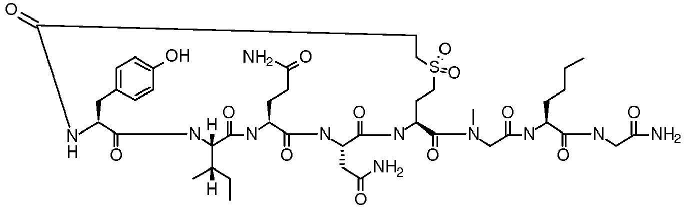

The following amino acids were used: Fmoc-Gly-OH, Fmoc-Nle-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 980.1; observed 980.4

### Example 10

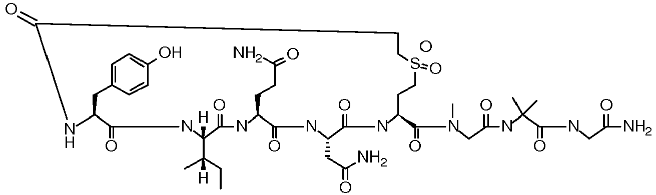

The following amino acids were used: Fmoc-Gly-OH, Fmoc-Aib-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 952.0; observed 952.4

### Example 11

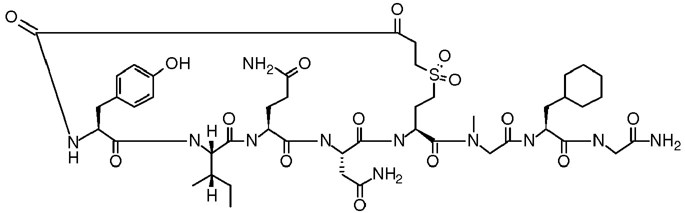

The following amino acids were used: Fmoc-Gly-OH, Fmoc-Cha-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1020.2; observed 1020.6

### Example 12

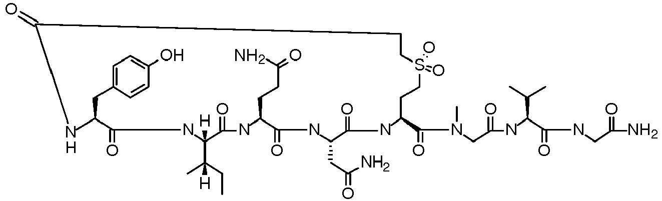

The following amino acids were used: Fmoc-Gly-OH, Fmoc-Val-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 966.1; observed 966.5

### Example 13

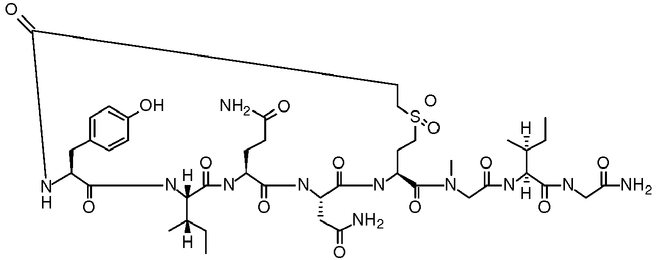

The following amino acids were used: Fmoc-Gly-OH, Fmoc-Ile-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 980.1; observed 980.4

### Example 14

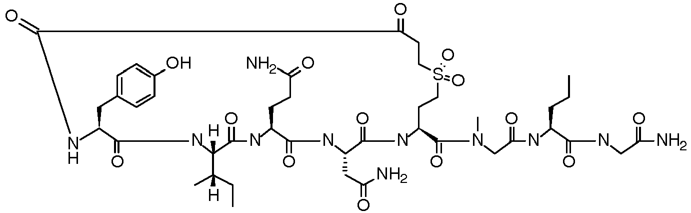

The following amino acids were used: Fmoc-Gly-OH, Fmoc-Nva-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 966.1; observed 966.5

### Example 15

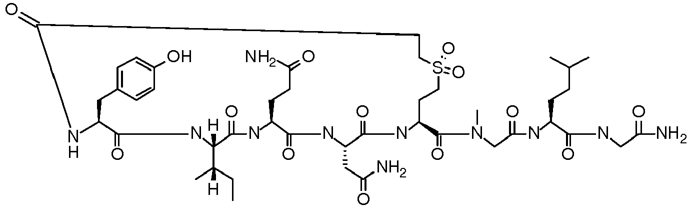

The following amino acids were used: Fmoc-Gly-OH, Fmoc-homoVal-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 994.1; observed 994.5

### Example 16

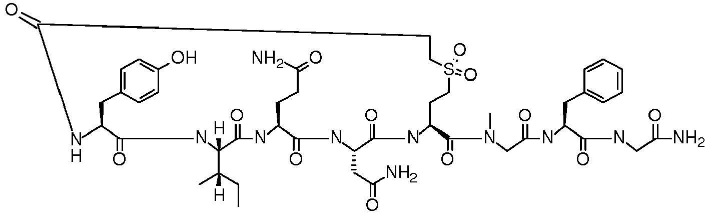

The following amino acids were used: Fmoc-Gly-OH, Fmoc-Phe-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1014.1; observed 1014.4

### Example 17

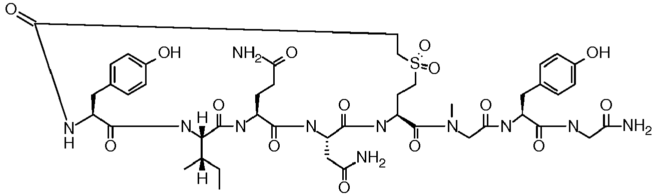

The following amino acids were used: Fmoc-Gly-OH, Fmoc-Tyr(tBu)-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1030.1; observed 1030.4

### Example 18

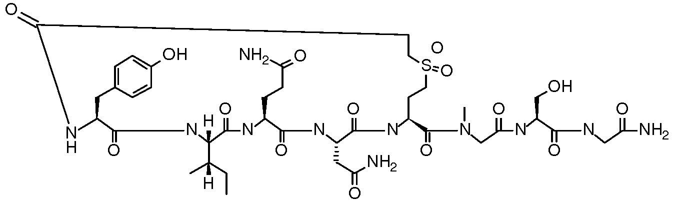

The following amino acids were used: Fmoc-Gly-OH, Fmoc-Ser(tBu)-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 954.0; observed 954.4

### Example 19

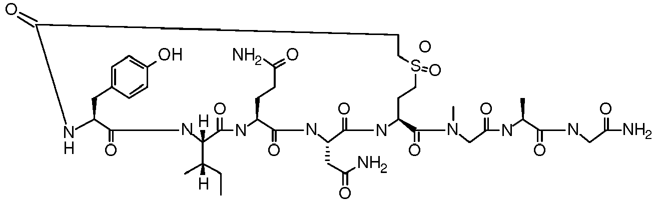

The following amino acids were used: Fmoc-Gly-OH, Fmoc-Ala-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 938.0; observed 938.4

### Example 20

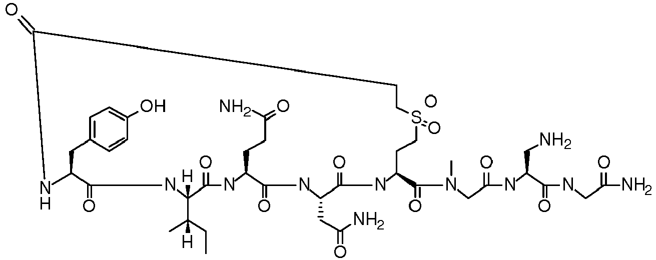

The following amino acids were used: Fmoc-Gly-OH, Fmoc-Dap(BOC)-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 953.0; observed 953.4

### Example 21

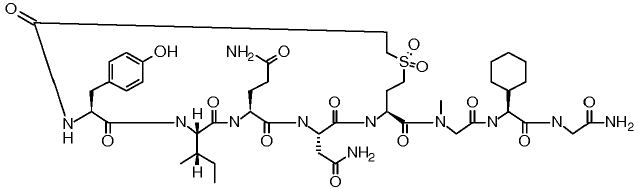

The following amino acids were used: Fmoc-Gly-OH, Fmoc-Chg-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1006.1; observed 1006.4

### Example 22

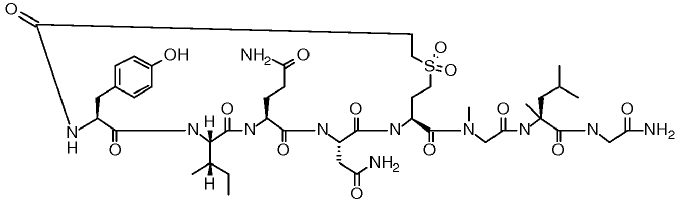

The following amino acids were used: Fmoc-Gly-OH, Fmoc- -MeLeu-OH, Fmoc-Sar-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 994.1; observed 994.5

### Example 23

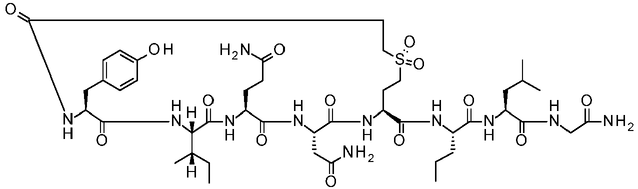

The following amino acids were used: Fmoc-Gly-OH, Fmoc-Leu-OH, Fmoc-Nva-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1008.1; observed 1008.4

### Example 24

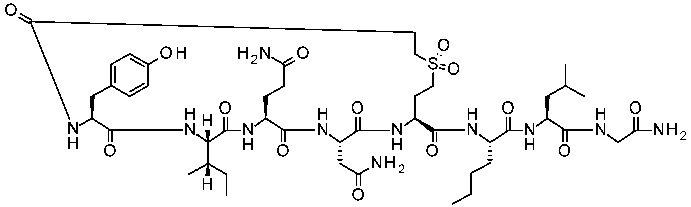

The following amino acids were used: Fmoc-Gly-OH, Fmoc-Leu-OH, Fmoc-Nle-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1022.2; observed 1022.4

### Example 25

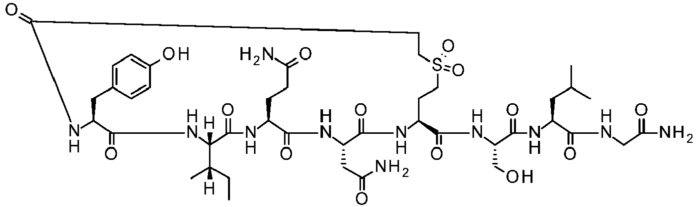

The following amino acids were used: Fmoc-Gly-OH, Fmoc-Leu-OH, Fmoc-Ser(tBu)-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 996.1; observed 996.3

### Example 26

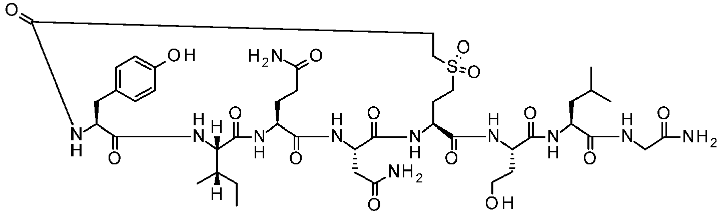

The following amino acids were used: Fmoc-Gly-OH, Fmoc-Leu-OH, Fmoc-homoSer(tBu)-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1010.1; observed 1010.4

### Example 27

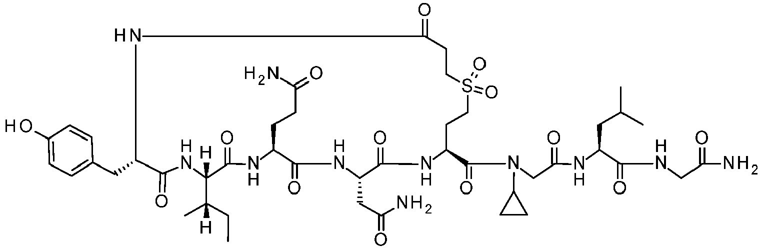

The following amino acids were used: Fmoc-Gly-OH, Fmoc-Leu-OH, Fmoc-CyclopropGly-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1006.1; observed 1006.4

### Example 28

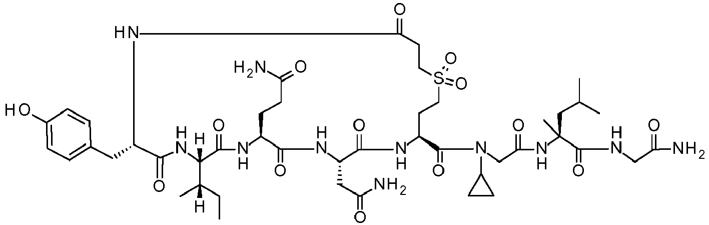

The following amino acids were used: Fmoc-Gly-OH, Fmoc-a-MeLeu-OH, Fmoc-CyclopropGly-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1020.1; observed 1020.5

### Example 29

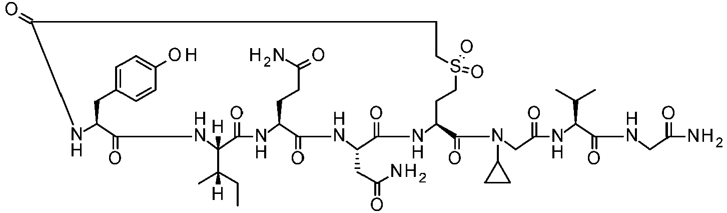

The following amino acids were used: Fmoc-Gly-OH, Fmoc-Val-OH, Fmoc-CyclopropGly-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 992.1; observed 992.5

### Example 30

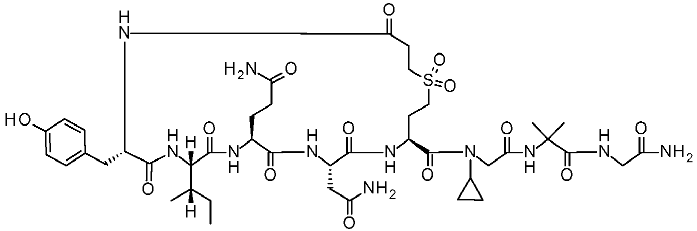

The following amino acids were used: Fmoc-Gly-OH, Fmoc-Aib-OH, Fmoc-CyclopropGly-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 978.1; observed 978.5

### Example 31

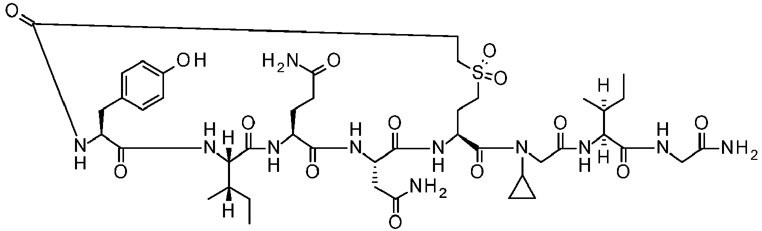

The following amino acids were used: Fmoc-Gly-OH, Fmoc-Ile-OH, Fmoc-CyclopropGly-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1006.1; observed 1006.4

### Example 32

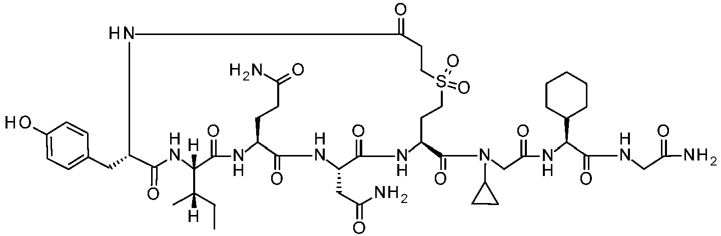

The following amino acids were used: Fmoc-Gly-OH, Fmoc-Chg-OH, Fmoc-CyclopropGly-OH, Fmoc-(S)-2-Amino-4-(2-tert-butoxycarbonyl-ethanesulfonyl)-butyric acid, Fmoc-Asn(Trt)-OH, Fmoc-Gln(Trt)-OH, Fmoc-Ile-OH and Fmoc-Tyr(tBu)-OH.
MS (M+H⁺): expected 1032.2; observed 1032.4

### Material and Methodes

### Cell culture and stable clone production

Chines Hamster Ovary (CHO) cells were transfected with expression plasmids encoding either the human V1a, the human Oxytocin (OTR) or the humanV2 receptor, the later in combination with the chimeric Gqs5 G protein to redirect the signal to Calcium flux. Stable cells were cloned by limiting dilution to yield monoclonal cell lines expressing either human V1a, human V2+Gqs5 or human OTR receptors and selected based on functional responses detected on a fluorometric imaging plate reader (FLIPR) detecting Calcium flux in the cell after receptor activation. The stable cell lines were grown in F-12 K Nutrient Mixture (Kaighns Modification), containing 10 % foetal bovine serum (FBS), 1 % penicillin-streptomycin, 1 % L-glutamate, 200 ug/ml Geneticin at 37 °C in a 10 % CO₂ incubator at 95 % humidity.

### Calcium flux assays using fluorescent imaging (Fluorometric Imaging Plate Reader, FLIPR)

On the afternoon before the assay, cells were plated at a density of 50,000 cells/well into black 96 well plates with clear bottoms to allow cell inspection and fluorescence measurements from the bottom of each well. The density of cells was sufficient to yield a confluent monolayer the next day. Hanks balanced salt solution, without phenol red, containing 20 mM HEPES (pH 7.3) and 2.5 mM probenecid (assay buffer) was prepared fresh for each experiment. Compound dilutions were made using a Beckman Biomek 2000 laboratory automation workstation, in assay buffer containing 1 % DMSO. The dye-loading buffer consisted of a final concentration of 2 µM Fluo-4-AM (dissolved in DMSO and pluronic acid) in assay buffer. The existing culture media was removed from the wells and 100 µl of the dye-loading buffer was added to each well and incubated for approximately 60 min at 37 °C in a 5 % CO₂ incubator at 95 % humidity. Once dye-loaded, the cells were washed thoroughly on an Embla cell washer with the assay buffer to remove any unincorporated dye. Exactly 100 µl assay buffer was left in each well.

Each 96 well plate containing dye-loaded cells was placed into the FLIPR machine and the laser intensity set to a suitable level to detect low basal fluorescence. To test compounds as agonists, 25 µl diluted compound was added to the plate 10 seconds into the fluorescent measurements and fluorescent response was recorded for 5 minutes. The fluorescence data was normalized to the endogenous full agonist dose-response set at 100 % for the maximum response and 0 % for the minimum. Each agonist concentration-response curve was constructed using a four parameter logistic equation with Microsoft Excel XLFit as follows: Y = Minimum + ((Maximum - Minimum) / (1 + 10 ^{(LogEC50-X)nH})), where y is the % normalized fluorescence, minimum is the minimum y, maximum is the maximum y, logEC₅₀ is the log₁₀ concentration which produces 50 % of the maximum induced fluorescence, x is the log₁₀ of the concentration of the agonist compound and H is the slope of the curve (the Hill Coefficient). The maximum value gives the efficacy of the agonist test compound in percentage. The concentration of agonist that produced a half-maximal response is represented by the EC₅₀ value, the logarithm of which yielded the pEC₅₀ value.

**The following EC₅₀ (nM), and efficacy (%) for the specific peptides may be provided, together with comparative data for hV1a and hV2:**

| **Expl.** | **hOT EC₅₀(nM) / efficacy (%)** | **hV1a EC₅₀ (nM)** | **hV2 EC₅₀ (nM)/ efficacy (%)** | **Expl.** | **hOT EC₅₀(nM)/ efficacy (%)** | **hV1a EC₅₀ (nM)/ efficacy (%)** | **hV2 EC₅₀ (nM) efficacy (%)** |
|---|---|---|---|---|---|---|---|
| 1 | 0.8/120 | 1633 | 2982/101 | 18 | 3.3/102 | | |
| 2 | 0.8/107 | >27000 | 3984/86 | 19 | 1.4/110 | | |
| 3 | 1.7/144 | | | 20 | 12.9/113 | | |
| 4 | 3.0/128 | | | 21 | 0.4/117 | | |
| 5 | 3.8/130 | | | 22 | 2.5/109 | | |
| 6 | 1.6/145 | | | 23 | 23/123 | | |
| 7 | 2.6/141 | | | 24 | 40/114 | | |
| 8 | 0.8/129 | | | 25 | 16/137 | | |
| 9 | 1.0/104 | | | 26 | 7/140 | | |
| 10 | 4.7/100 | | | 27 | 0.5/125 | >27000 | 3730/110 |
| 11 | 1.4/115 | | | 28 | 0.7/105 | >27000 | 9423/129 |
| 12 | 1.4/112 | | | 29 | 0.5/105 | | 3344/143 |
| 13 | 1.1/117 | | | 30 | 1/97 | | 6339/131 |
| 14 | 0.7/117 | | | 31 | 0.4/115 | | 2942/150 |
| 15 | 1.0/107 | | | 32 | 0.26/120 | 2233/38 | 2365/155 |
| 16 | 5.6/104 | | | | | | |
| 17 | 2.2/105 | | | | | | |

The compounds of formula I and the pharmaceutically acceptable salts of the compounds of formula I can be used as medicaments, e.g. in the form of pharmaceutical preparations. The pharmaceutical preparations can be administered preferably transdermal, intranasal, subcutaneous or intra venous (iv).

Transdermal is a route of administration wherein active ingredients are delivered across the skin for systematic distribution. Examples include transdermal patches used for medicine delivery, and transdermal implants used for medical or aesthetic purposes.

Nasal administration can be used to deliver drugs for either local or systemic effects, nasal sprays for local effect are quite common. Peptide drugs may be administered as nasal sprays to avoid drug degradation after oral administration.

Subcutaneous injections are also common for the administration of peptide drugs. An intramuscular injection is the injection of a substance directly into the muscle. It is one of several alternative methods for the administration of medications. It is often used for particular forms of medication that are administered in small amounts. The injections should be given under the skin.

The intravenous route is the infusion of liquid substances directly into a vein. Compared with other routes of administration, the intravenous route is the fastest way to deliver fluids and medications throughout the body.

The pharmaceutical preparations can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or a pharmaceutically acceptable salt thereof and a therapeutically inert carrier are also an object of the present invention, as is a process for their production, which comprises bringing one or more compounds of formula I and/or pharmaceutically acceptable acid addition salts and, if desired, one or more other therapeutically valuable substances into a galenical administration form together with one or more therapeutically inert carriers.
The most preferred indications in accordance with the present invention are those which include disorders of the central nervous system, for example the treatment or prevention of autism, stress, including post traumatic stress disorder, anxiety, including anxiety disorders and depression, schizophrenia, psychiatric disorders and memory, loss alcohol withdrawel, drug addiction and for the treatment of Prader-Willi Syndrom..

The dosage can vary within wide limits and will, of course, have to be adjusted to the individual requirements in each particular case. The dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

## Claims

1. A compound of formula 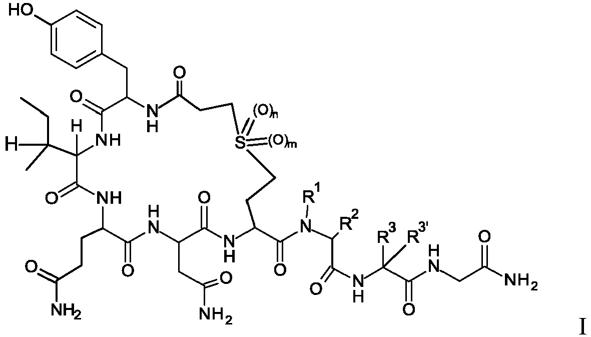 wherein
R¹ is hydrogen, C₁₋₇-alkyl, -CH₂-C₃₋₆-cycloalkyl or C₃₋₆-cycloalkyl;
R² is hydrogen, C₁₋₇-alkyl, C₁₋₇-alkyl substituted by hydroxy or
R¹ and R² may form together with the N and C atom to which they are attached a pyrrolidine ring optionally substituted by one or two F-atoms or by hydroxy, or may form an azetidine or a piperidine ring;
R³ is hydrogen, C₁₋₇-alkyl, C₁₋₇-alkyl substituted by hydroxy, -(CH₂)ₒNH₂, benzyl optionally substituted by hydroxy, phenyl, -CH₂-C₃₋₆-cycloalkyl or C₃₋₆-cycloalkyl;
R^{3'} is hydrogen or C₁₋₇-alkyl;
n is 1;
m is 0 or 1;
o is 1 to 4;
or a pharmaceutically acceptable acid addition salt, a racemic mixture or its corresponding enantiomer and/or optical isomers thereof.

2. A compound of formula I according to claim 1, wherein m is 1.

3. A compound of formula I according to any one of claims 1 or 2, wherein R¹ is hydrogen, C₁₋₇-alkyl, -CH₂-C₃₋₆-cycloalkyl or C₃₋₆-cycloalkyl and R² is hydrogen, C₁₋₇-alkyl, C₁₋₇-alkyl substituted by hydroxy and the other definitions are as described in claim 1.

4. A compound of formula I according to any one of claims 1 or 2, wherein R¹ and R² may form together with the N and C atom to which they are attached a pyrrolidine ring optionally substituted by one or two F-atoms or by hydroxy, or may form an azetidine or a piperidine ring, and the other definitions are as described in claim 1.

5. A compound of formula I accoccording to claim 1, wherein the compounds are

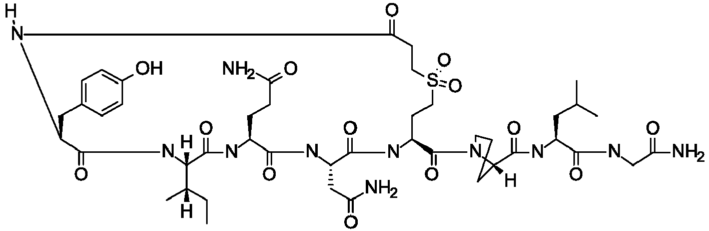

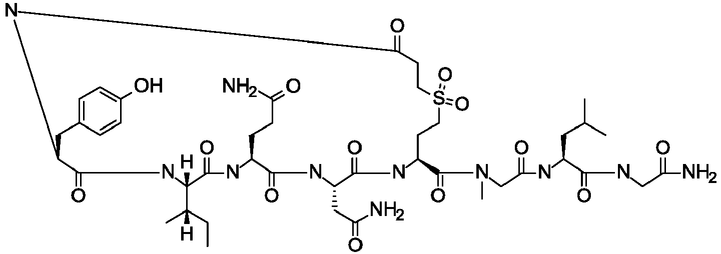

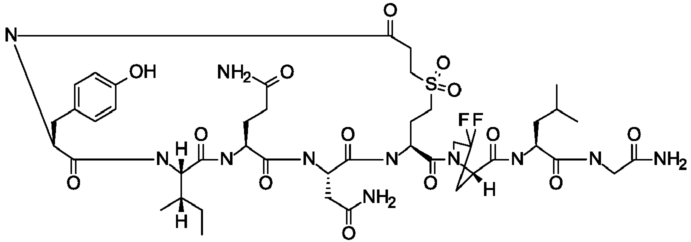

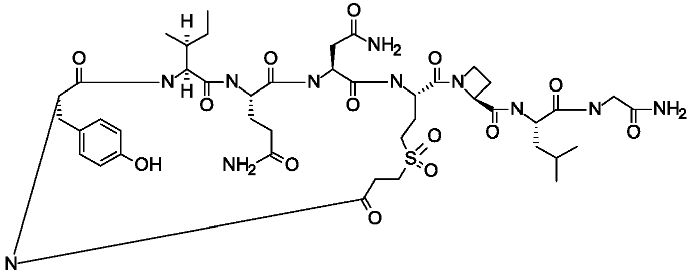

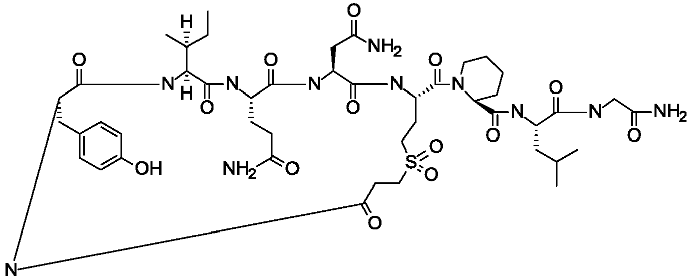

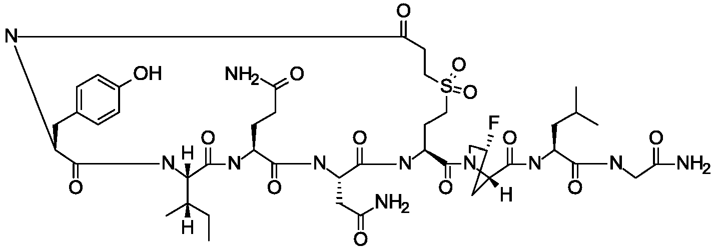

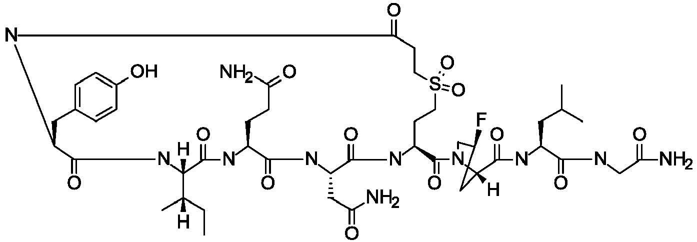

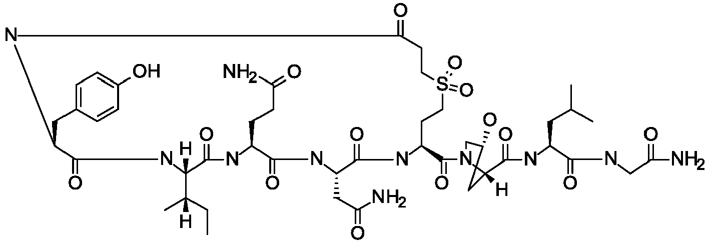

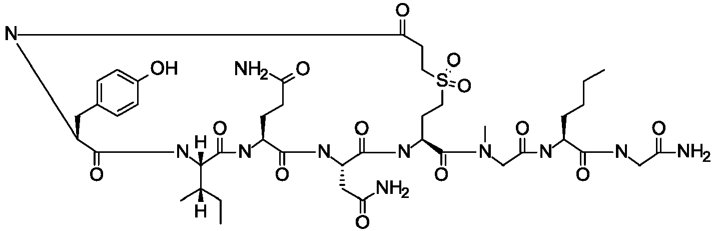

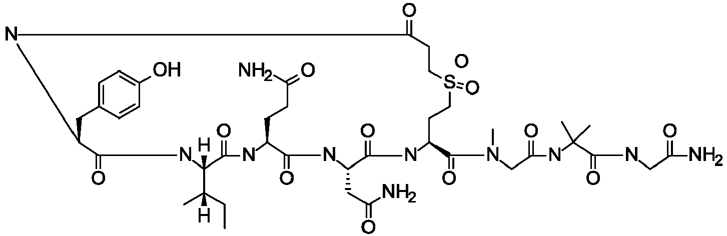

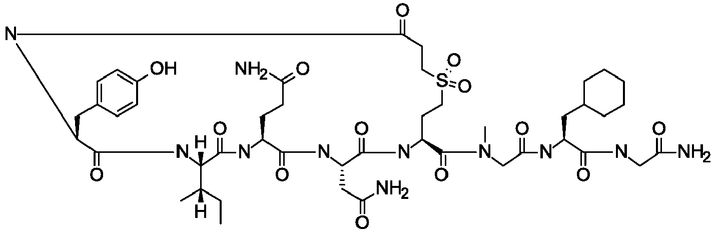

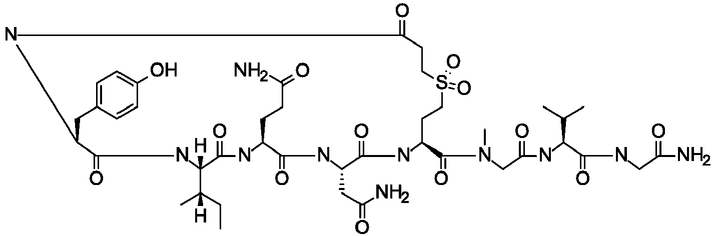

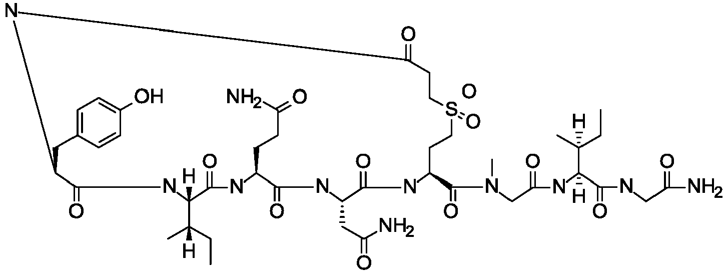

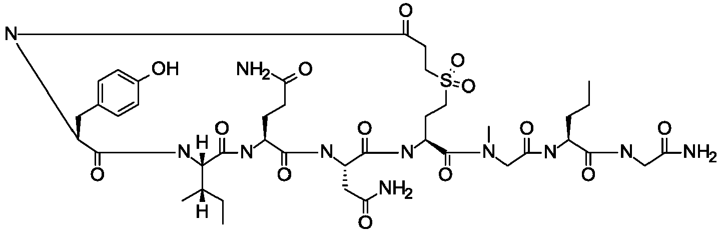

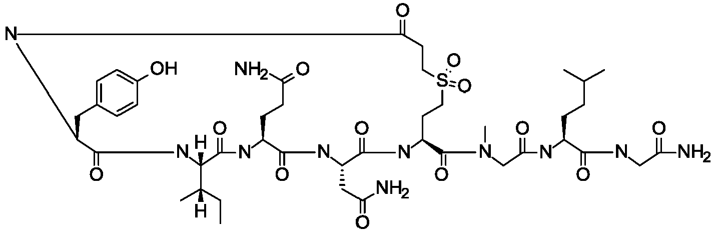

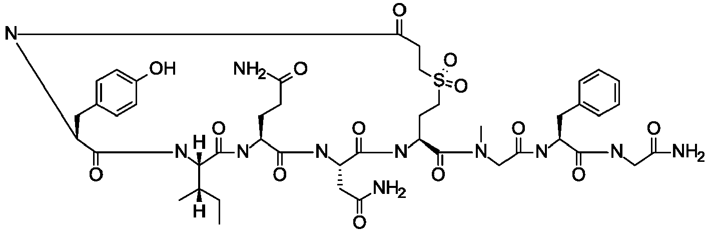

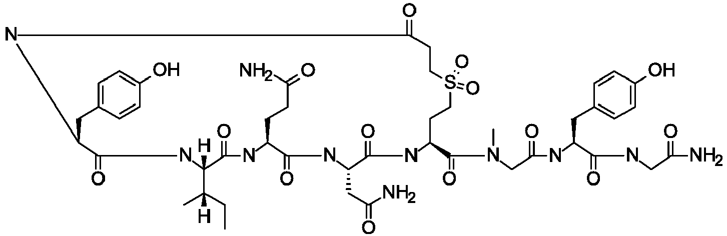

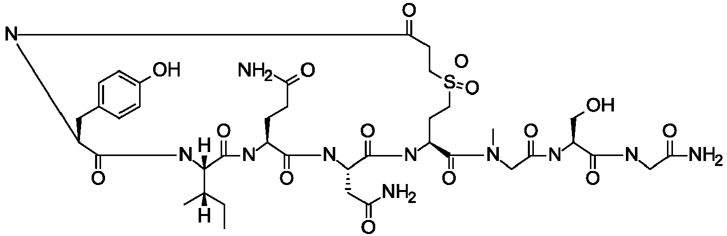

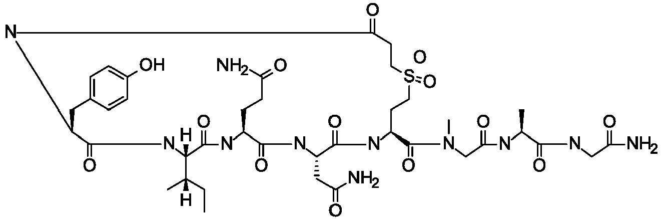

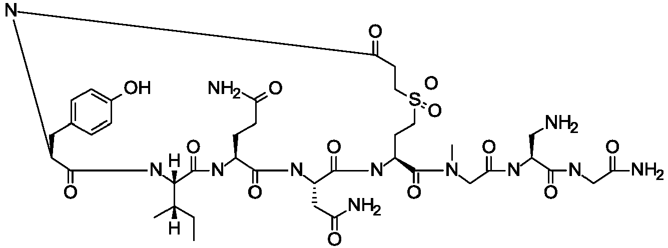

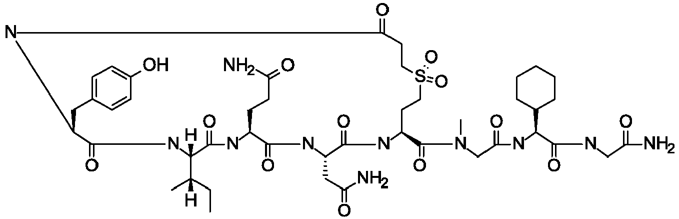

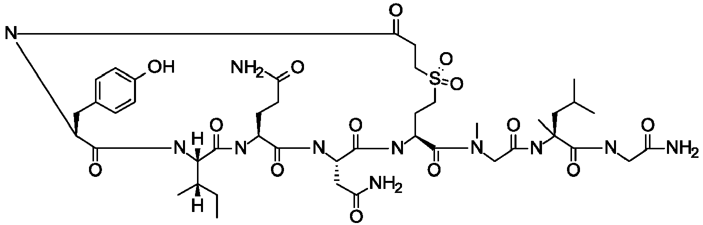

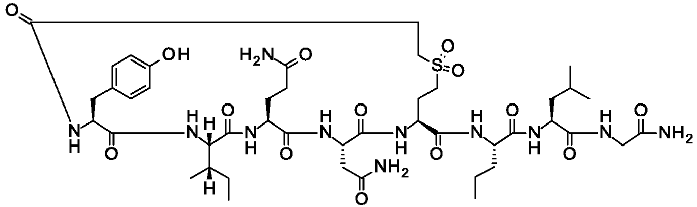

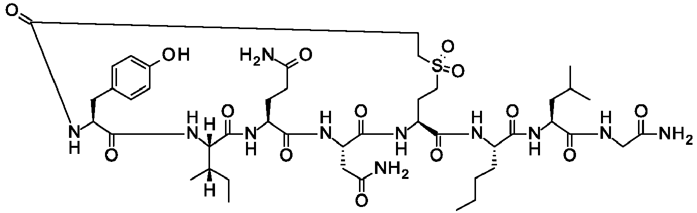

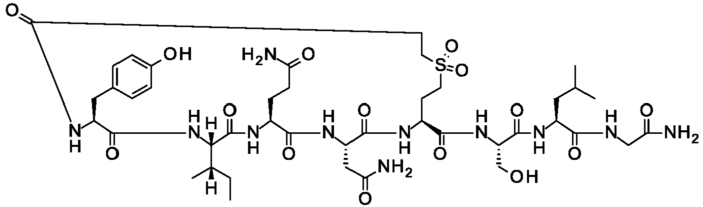

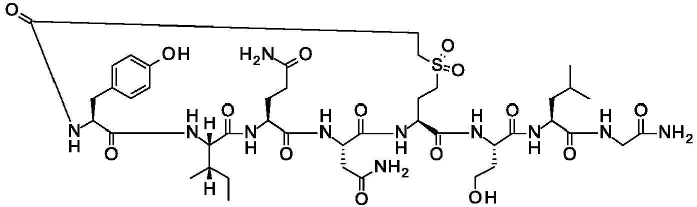

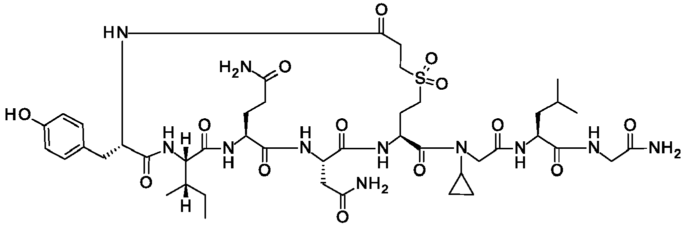

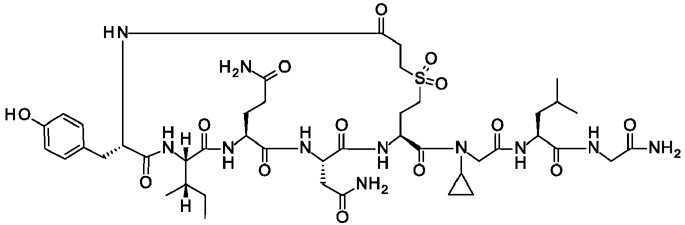

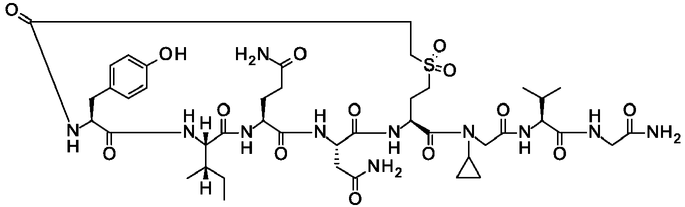

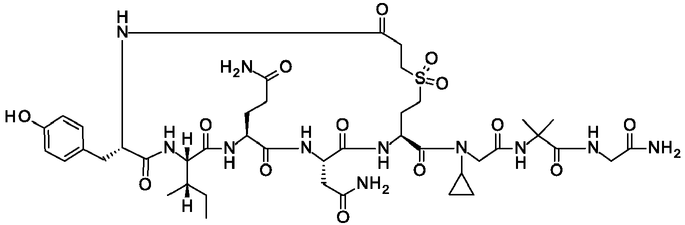

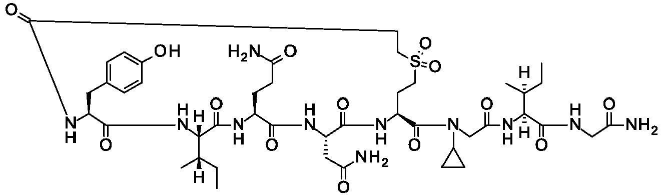

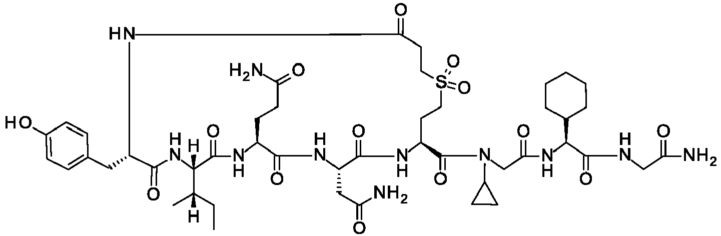

6. A pharmaceutical composition comprising a compound according to any one of claims 1 -5 and a pharmaceutical acceptable carrier and/or adjuvant.

7. A pharmaceutical composition comprising a compound according to any one of claims 1-5 and a pharmaceutical acceptable carrier and/or adjuvant for use in the treatment of autism, stress, including post traumatic stress disorder, anxiety, including anxiety disorders and depression, schizophrenia, psychiatric disorders and memory loss, alcohol withdrawel, drug addiction and for the treatment of Prader-Willi Syndrom.

8. A compound according to any one of claims 1-5 for use as therapeutic active substances.

9. A compound according to any one of claims 1-5 for use as therapeutic active substances in the treatment of autism, stress, including post traumatic stress disorder, anxiety, including anxiety disorders and depression, schizophrenia, psychiatric disorders and memory loss, alcohol withdrawel, drug addiction and for the treatment of Prader Willi Syndrom.

## Patentansprüche

1. Verbindung der Formel 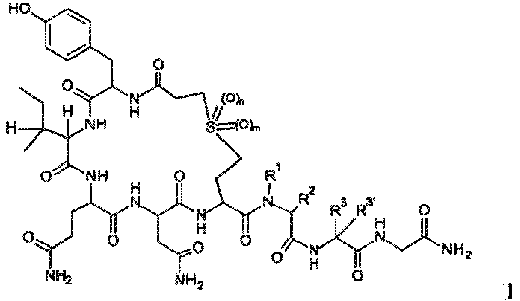 wobei
R¹ Wasserstoff, C₁₋₇-Alkyl, -CH₂-C₃₋₆-Cycloalkyl oder C₃₋₆-Cycloalkyl ist;
R² Wasserstoff, C₁₋₇-Alkyl, mit Hydroxy substituiertes C₁₋₇-Alkyl ist, oder
R¹ und R² zusammen mit dem N- und C-Atom, an das sie gebunden sind, einen Pyrrolidinring bilden können, der optional mit ein oder zwei F-Atomen oder mit Hydroxy substituiert ist, oder einen Azetidin- oder Piperidinring bilden können;
R³ Wasserstoff, C₁₋₇-Alkyl, mit Hydroxy substituiertes C₁₋₇-Alkyl, -(CH₂)ₒNH₂, optional mit Hydroxy substituiertes Benzyl, Phenyl, -CH₂-C₃₋₆-Cycloalkyl oder C₃₋₆-Cycloalkyl ist;
R^{3'} Wasserstoff oder C₁₋₇-Alkyl ist;
n 1 ist;
m 0 oder 1 ist;
o 1 bis 4 ist;
oder ein pharmazeutisch unbedenkliches Säureadditionssalz, eine racemische Mischung oder dessen entsprechendes Enantiomer und/oder optische Isomere davon.

2. Verbindung der Formel I nach Anspruch 1, wobei m 1 ist.

3. Verbindung der Formel I nach einem der Ansprüche 1 oder 2, wobei R¹ Wasserstoff, C₁₋₇-Alkyl, -CH₂-C₃₋₆-Cycloalkyl oder C₃₋₆-Cycloalkyl ist und R² Wasserstoff, C₁₋₇-Alkyl, mit Hydroxy substituiertes C₁₋₇-Alkyl ist, und die anderen Definitionen wie in Anspruch 1 beschrieben sind.

4. Verbindung der Formel I nach einem der Ansprüche 1 oder 2, wobei R¹ und R² zusammen mit dem N- und C-Atom, an das sie gebunden sind, einen Pyrrolidinring bilden können, der optional mit ein oder zwei F-Atomen oder mit Hydroxy substituiert ist, oder einen Azetidin- oder Piperidinring bilden können, und die anderen Definitionen wie in Anspruch 1 beschrieben sind.

5. Verbindung der Formel I nach Anspruch 1, wobei die Verbindungen Folgende sind

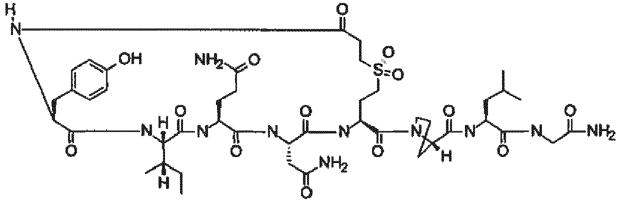

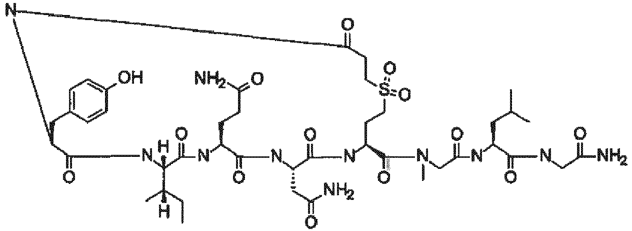

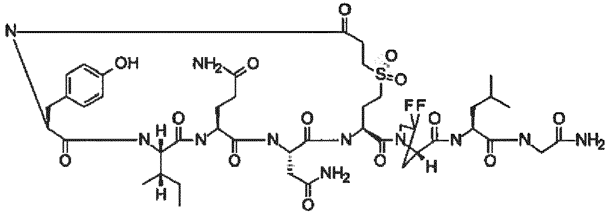

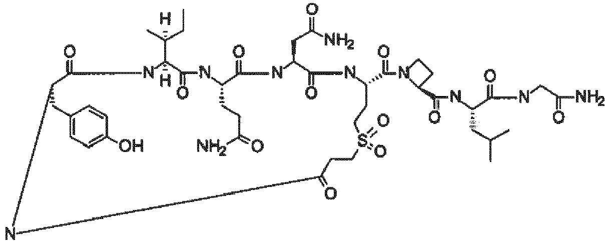

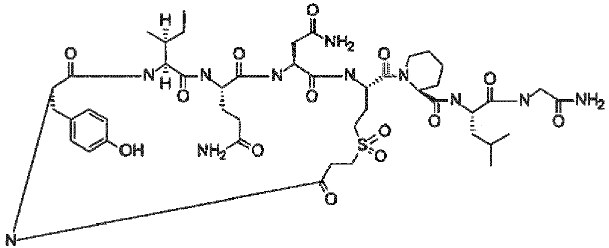

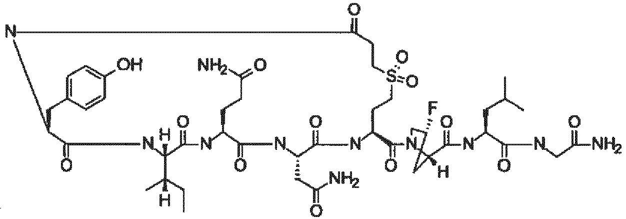

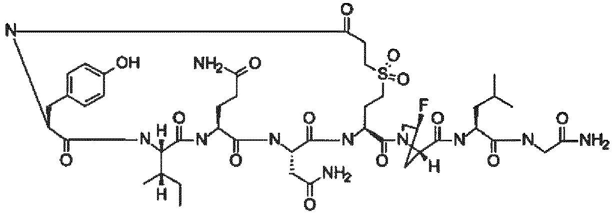

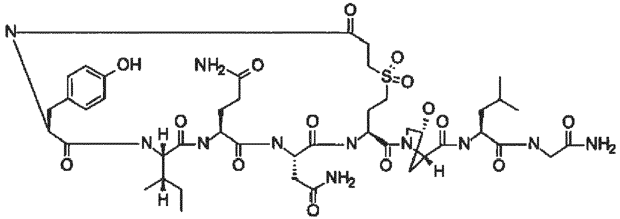

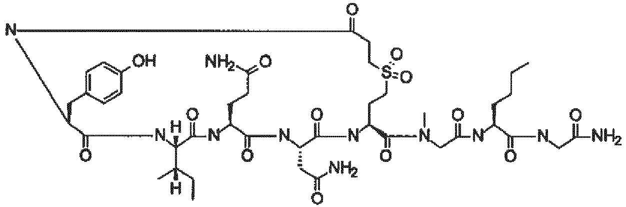

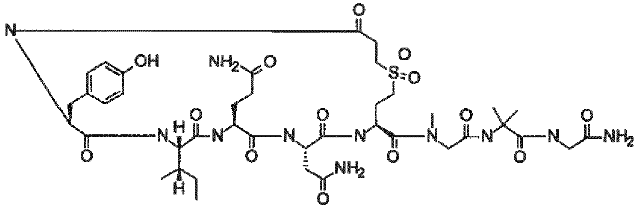

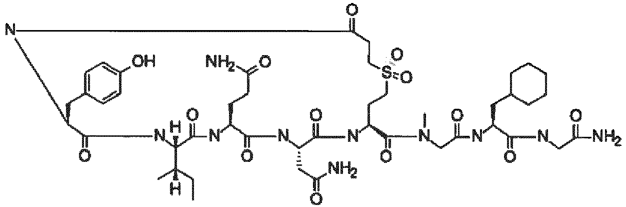

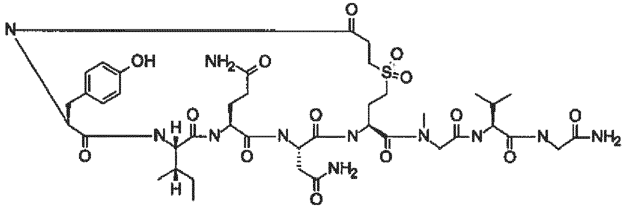

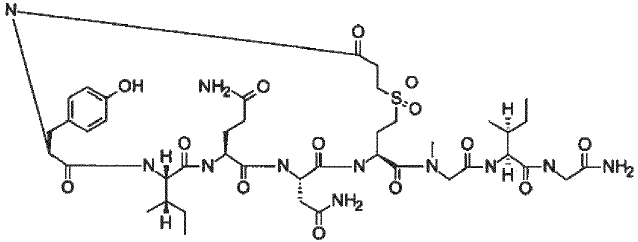

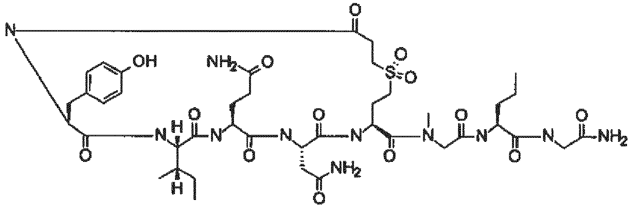

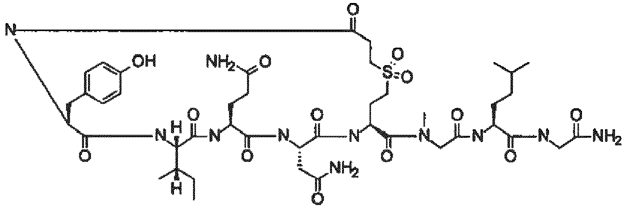

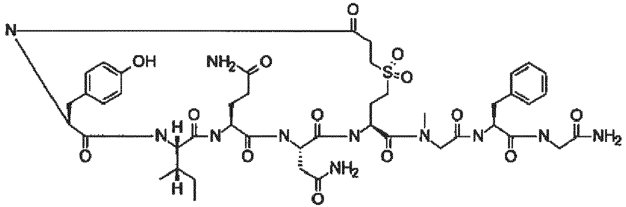

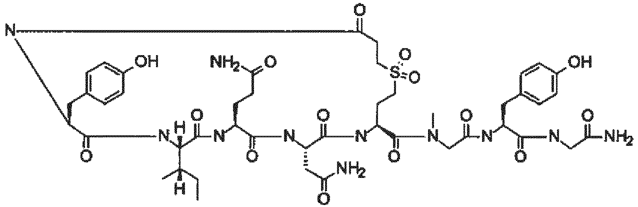

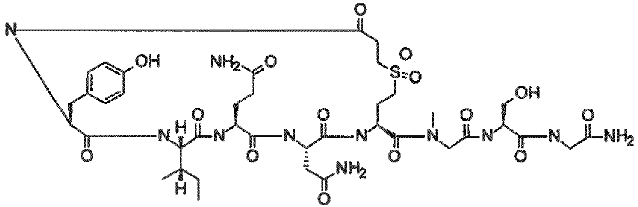

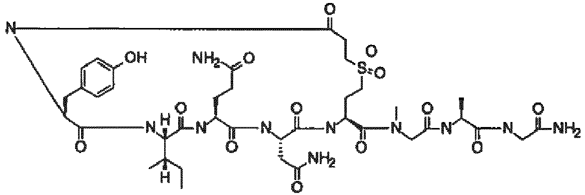

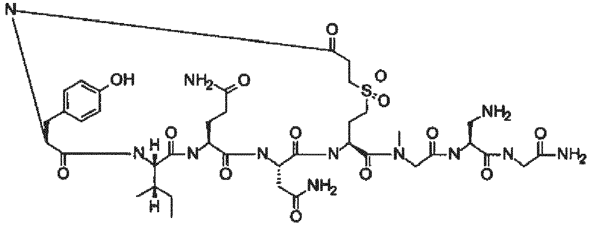

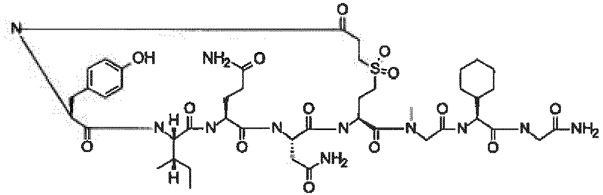

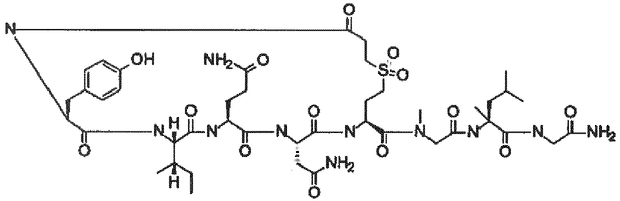

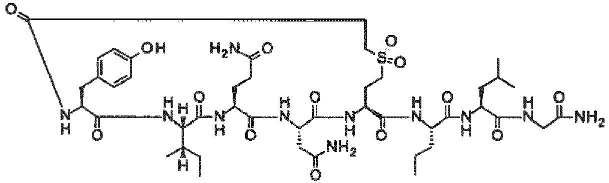

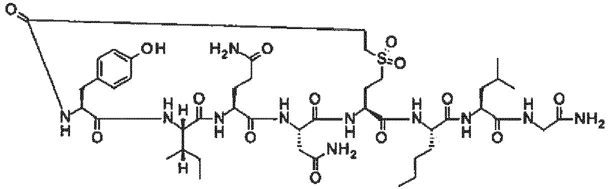

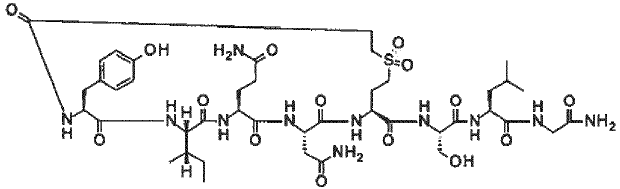

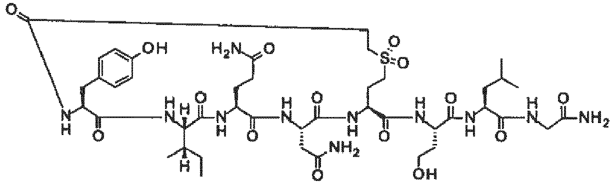

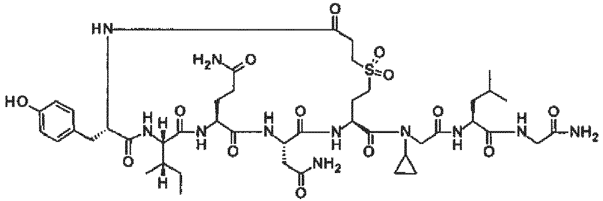

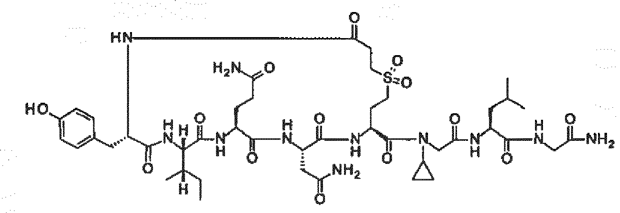

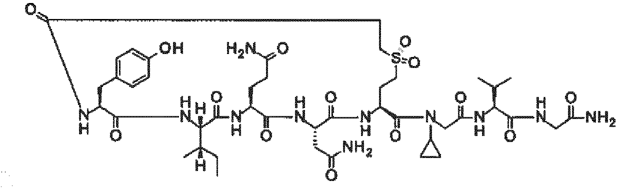

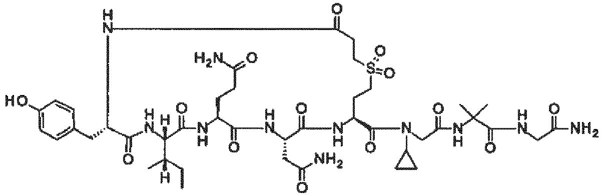

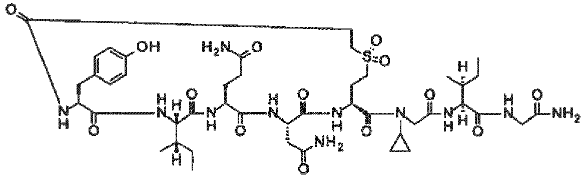

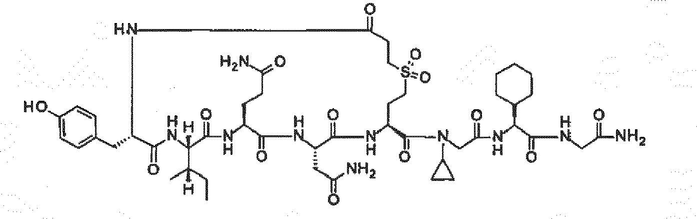

6. Pharmazeutische Zusammensetzung, umfassend eine Verbindung nach einem der Ansprüche 1-5 und einen pharmazeutisch unbedenklichen Träger und/oder ein pharmazeutisch unbedenkliches Adjuvans.

7. Pharmazeutische Zusammensetzung, umfassend eine Verbindung nach einem der Ansprüche 1-5 und einen pharmazeutisch unbedenklichen Träger und/oder ein pharmazeutisch unbedenkliches Adjuvans, zur Verwendung bei der Behandlung von Autismus, Stress, einschließlich posttraumatischer Belastungsstörung, Angst, einschließlich Angststörungen und Depression, Schizophrenie, psychiatrischen Störungen und Gedächtnisverlust, Alkoholentzug, Drogenabhängigkeit und zur Behandlung des Prader-Willi-Syndroms.

8. Verbindung nach einem der Ansprüche 1-5 zur Verwendung als therapeutische Wirkstoffe.

9. Verbindung nach einem der Ansprüche 1-5 zur Verwendung als therapeutische Wirkstoffe bei der Behandlung von Autismus, Stress, einschließlich posttraumatischer Belastungsstörung, Angst, einschließlich Angststörungen und Depression, Schizophrenie, psychiatrischen Störungen und Gedächtnisverlust, Alkoholentzug, Drogenabhängigkeit und zur Behandlung des Prader-Willi-Syndroms.

## Revendications

1. Composé de formule 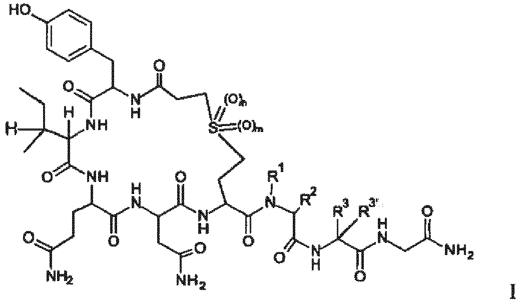 dans laquelle
R¹ représente un hydrogène, un alkyle en C₁₋₇, un CH₂-cycloalkyle en C₃₋₆ ou un cycloalkyle en C₃₋₆ ;
R² représente un hydrogène, un alkyle en C₁₋₇, un alkyle en C₁₋₇ substitué par un hydroxy ou
R¹ et R² peuvent former conjointement avec les atomes N et C auxquels ils sont fixés un cycle pyrrolidine, éventuellement substitué par un ou deux atomes de F ou par un hydroxy, ou peuvent former un cycle azétidine ou pipéridine ;
R³ représente un hydrogène, un alkyle en C₁₋₇, un alkyle en C₁₋₇ substitué par un hydroxy, un -(CH₂)ₒNH₂, un benzyle éventuellement substitué par un hydroxy, un phényle, un CH₂-cycloalkyle en C₃₋₆- ou un cycloalkyle en C₃₋₆ ;
R^{3'} représente un hydrogène ou un alkyle en C₁₋₇ ;
n vaut 1 ;
m vaut 0 ou 1 ;
o vaut 1 à 4 ;
ou un sel d'addition d'acide pharmaceutiquement acceptable, un mélange racémique ou son énantiomère correspondant et/ou ses isomères optiques correspondants.

2. Composé de formule 1 selon la revendication 1, dans laquelle m vaut 1.

3. Composé de formule 1 selon l'une quelconque des revendications 1 ou 2, dans laquelle R¹ représente un hydrogène, un alkyle en C₁₋₇, un CH₂-cycloalkyle en C₃₋₆- ou un cycloalkyle en C₃₋₆, et R² représente un hydrogène, un alkyle en C₁₋₇, un alkyle en C₁₋₇ substitué par un hydroxy et les autres définitions sont telles que décrites dans la revendication 1.

4. Composé de formule I selon l'une quelconque des revendications 1 ou 2, dans lequel R¹ et R² peuvent former conjointement avec les atomes N et C auxquels ils sont fixés un cycle pyrrolidine, éventuellement substitué par un ou deux atomes de F ou par un hydroxy, ou peuvent former un cycle azétidine ou pipéridine, et les autres définitions sont telles que décrites selon la revendication 1.

5. Composé de formule I selon la revendication 1, dans lequel les composés sont

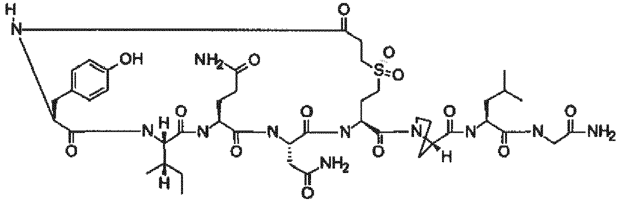

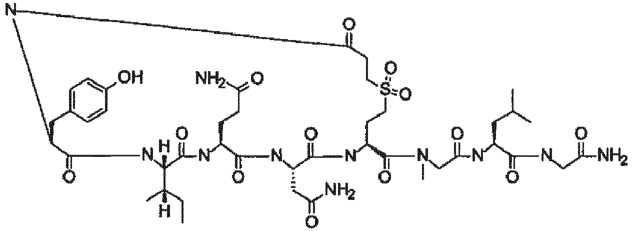

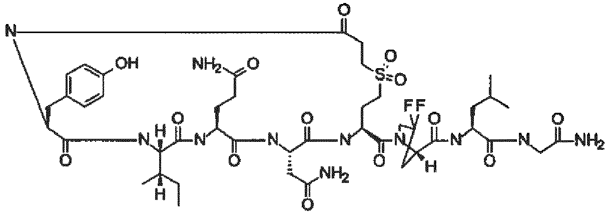

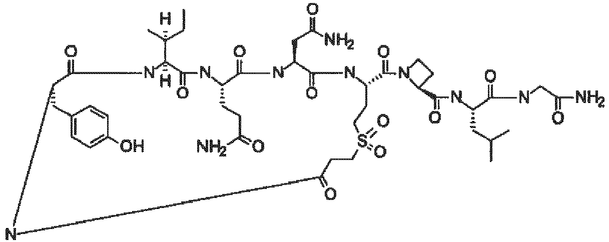

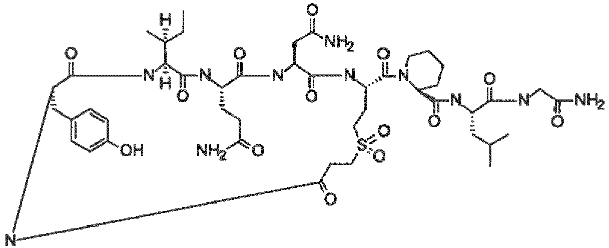

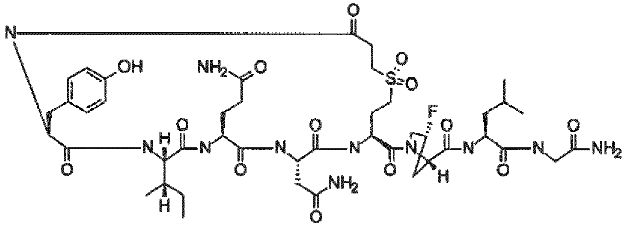

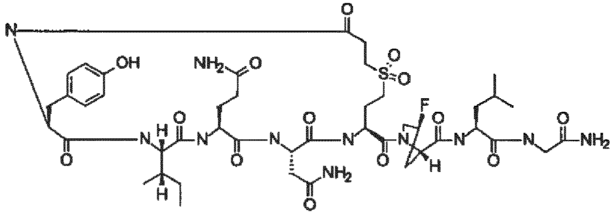

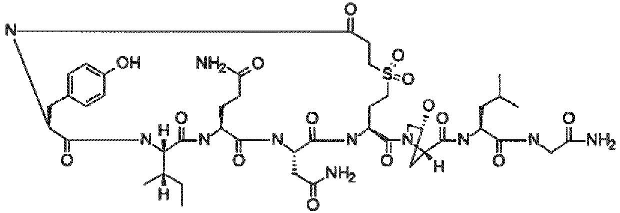

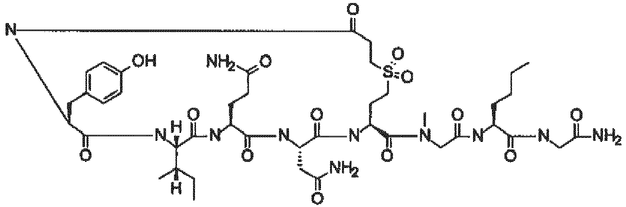

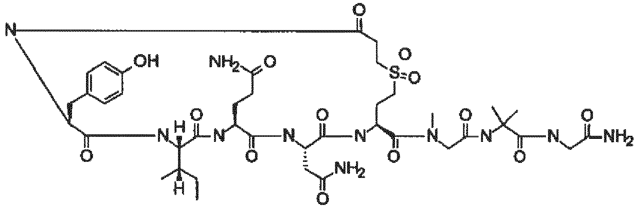

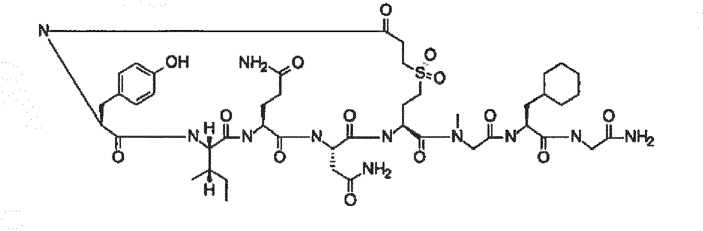

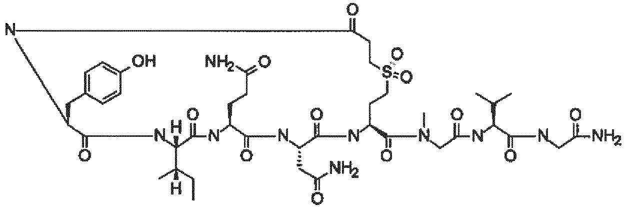

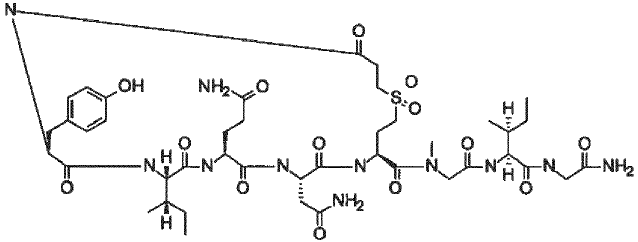

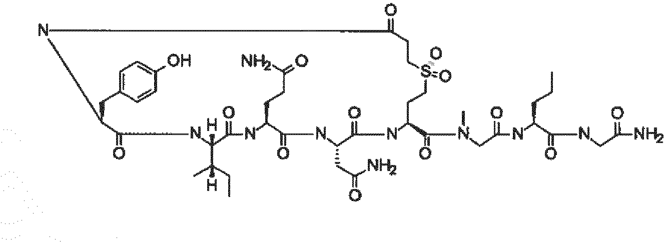

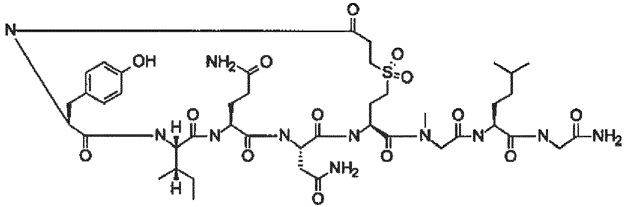

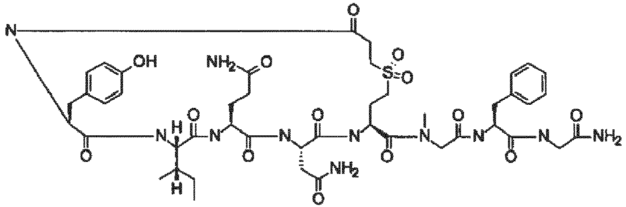

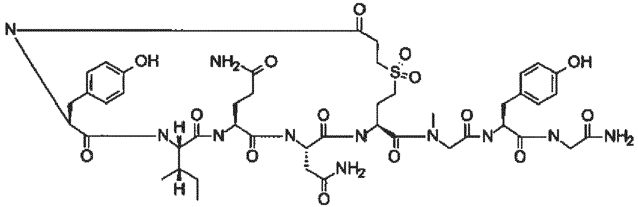

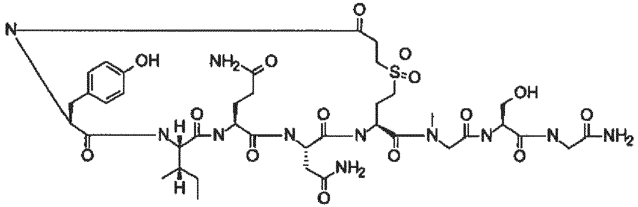

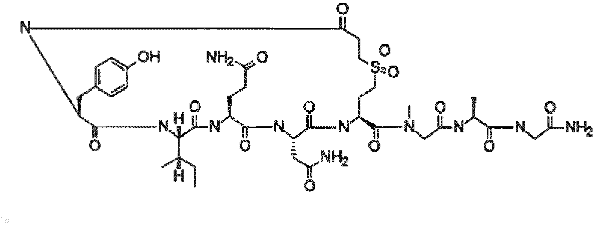

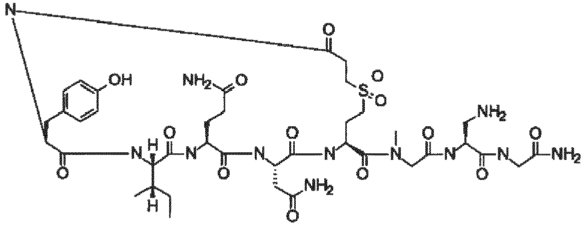

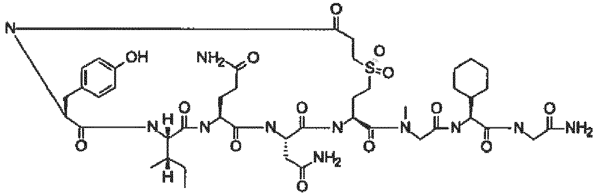

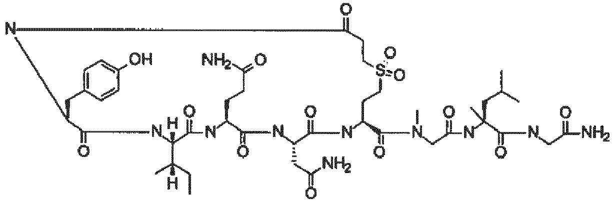

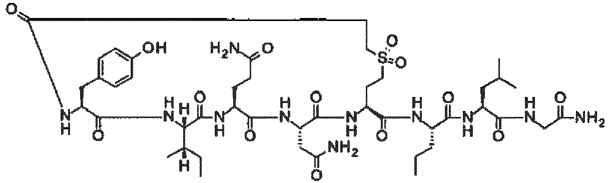

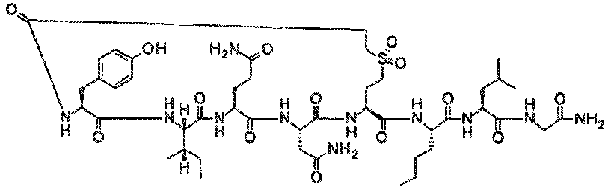

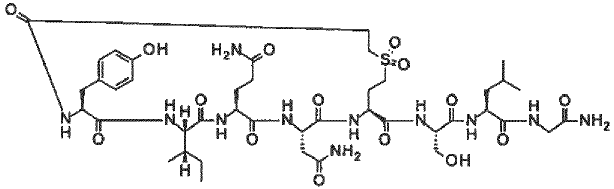

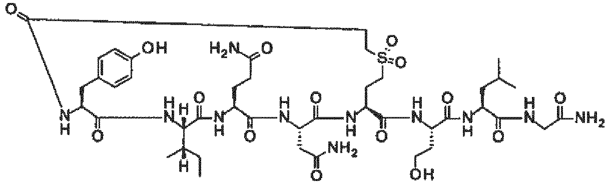

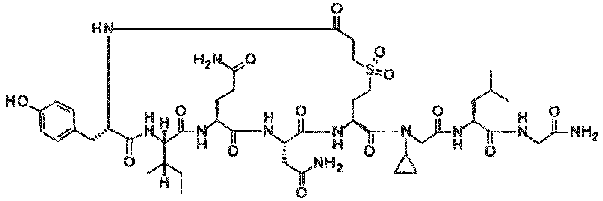

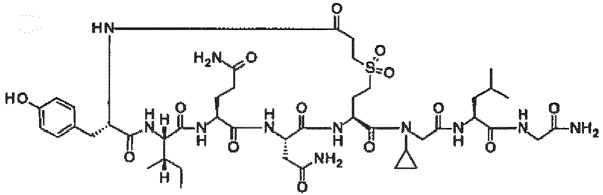

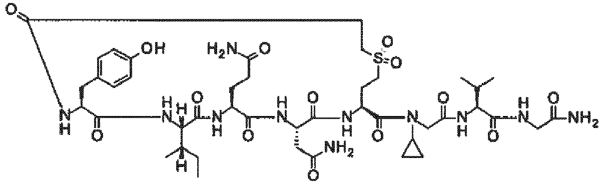

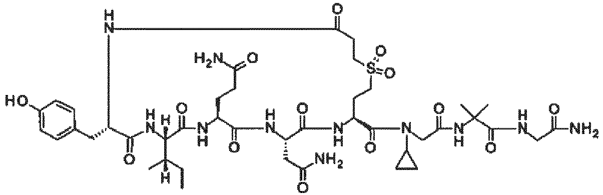

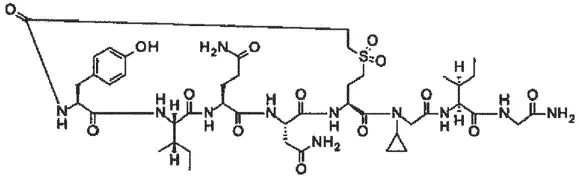

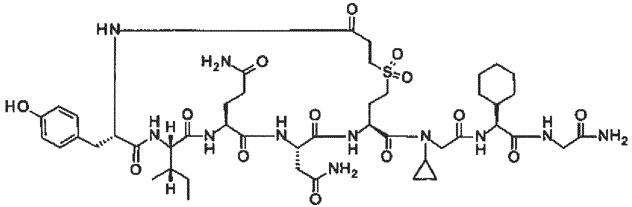

6. Composition pharmaceutique comprenant un composé selon l'une quelconque des revendications 1 à 5 et un vecteur et/ou un adjuvant pharmaceutiquement acceptables.

7. Composition pharmaceutique comprenant un composé selon l'une quelconque des revendications 1 à 5 et un vecteur et/ou un adjuvant pharmaceutiquement acceptables destinés à être utilisés dans le traitement de l'autisme, du stress, y compris le trouble de stress post-traumatique, de l'anxiété, y compris les troubles d'anxiété et la dépression, de la schizophrénie, des troubles psychiatriques et de la perte de mémoire, du sevrage alcoolique, de la toxicomanie et pour le traitement du syndrome de Prader-Willi.

8. Composé selon l'une quelconque des revendications 1 à 5 destiné à être utilisé en tant que substances thérapeutiquement actives.

9. Composé selon l'une quelconque des revendications 1 à 5 destiné à être utilisé en tant que substances thérapeutiquement actives dans le traitement de l'autisme, du stress, y compris le trouble de stress post-traumatique, de l'anxiété, y compris les troubles d'anxiété et la dépression, de la schizophrénie, des troubles psychiatriques et de la perte de mémoire, du sevrage alcoolique, de la toxicomanie et pour le traitement du syndrome de Prader-Willi.
